# EUROPEAN PATENT SPECIFICATION

(11) **EP 1 833 390 B1**
(45) Date of publication and mention of the grant of the patent: **12.05.2010**
(21) Application number: 05853387.8
(22) Date of filing: 08.12.2005
(51) Int. Cl.: A61B 17/32, A61B 17/16

(54) **WIRELESS SYSTEM FOR PROVIDING INSTRUMENT AND IMPLANT DATA TO A SURGICAL NAVIGATION UNIT**
DRAHTLOSES SYSTEM ZUR BEREITSTELLUNG VON INSTRUMENTEN- UND IMPLANTATDATEN AN EIN CHIRURGISCHES NAVIGATIONSGERÄT
SYSTEME SANS FIL POUR FOURNIR DES DONNEES SUR DES IMPLANTS ET INSTRUMENTS A UNE UNITE DE NAVIGATION CHIRURGICALE

(30) Priority: 09.12.2004 US 634588 P
(43) Date of publication of application: 19.09.2007
(73) Proprietor: STRYKER CORPORATION, Kalamazoo, MI 49002 (US)
(72) Inventor: MALACKOWSKI, Don, Schoolcraft, MI 49087 (US); LAVIGNA, Nicholas, Mountain Top, PA 18707 (US)
(74) Representative: Röthinger, Rainer
(86) International application number: PCT/US2005/044457
(87) International publication number: WO 2006/063156

(56) References cited:
- EP-A- 1 110 504
- WO-A-03/013372
- DE-A1- 10 239 673
- DE-A1- 10 241 070
- US-A1- 2003 093 103
- US-A1- 2004 092 991
- US-B1- 6 434 507

## Description

### FIELD OF THE INVENTION

This invention is generally directed to a surgical navigation system. More particularly, this invention is directed to a system for providing a surgical navigation system with information about the surgical instruments, surgical devices and implants tracked by the system without wired connections to the instruments, devices and implants.

### BACKGROUND OF THE INVENTION

Surgical navigation systems have become extremely useful tools in operating rooms. Generally, a surgical navigation system consists of one or more trackers. Each tracker is attached to a specific surgical instrument, device or implant. The system also includes a localizer. Each tracker generates one or more specific signals. These signals may be radio signals, other EM signals, light signals or ultrasonic signals. The localizer monitors from where the signals are broadcast. The tracker location-identifying signals obtained by the localizer are forwarded to a processor. Based on the data contained in these signals, including their signal strength and relative phases, the processor generates data indicating the position of the tracker and the attached component. Either prior to or at the start of the procedure, the processor is provided with data identifying the relative positions of tissue landmarks at the surgical site. The processor also has data that indicates the location of the surgical site relative to the localizer. Based on the above data, the processor provides information about the location of the surgical component attached to the tracker relative to the surgical site. Often this information is presented on a display that shows the position of the surgical component at a location within the patient. A surgical navigation system thus provides a view of the location of a surgical component at surgical site that, due to the presence of surrounding tissue, otherwise cannot be seen.

During the performance of a surgical procedure, a number of different instruments and other components are typically positioned at a surgical site. For example, during a procedure to implant an orthopedic implant, a first set of tissue cutting devices are used to gain access to the surgical site. A second set of devices are used to remove the bone and surrounding soft tissue that are to be replaced. A third set of devices shape the remaining tissue, typically the bone, so it can accept the implant. Often, trial implants are positioned at the surgical site to determine the appropriately sized implant components that should be permanently fitted to the patient. Then, the positions of the implant components themselves are tracked. Once an implant is fitted, the position of the instruments used to close the surgical site are tracked. Each of these instruments, implants and other components has a set of unique physical dimensions. For the surgical navigation system to accurately generate data indicating the , location of a component relative to the surgical site, the system processor must have data describing component's dimensions.

The Applicant's U.S. Patent Application No. 10/214,937, filed August 8, 2002, U.S. Patent Publication No. US 2003/0093103 A1, , discloses one system for providing a surgical navigation unit with data regarding the individual components applied to a surgical site. In the invention of this system, a radio frequency identification device (RFID) is attached to each component. Internal to the RFID is a memory in which data regarding the component are stored. These data identify the component and/or the physical dimensions of the component. The component also includes a coil through which the RFID is capable of exchanging signals. Prior to use, the component is attached to a handpiece. The handpiece is attached to some sort of control console. Internal to the handpiece is a coil. The handpiece coil and component coil are in sufficient proximity to allow inductive signal transfer. The control console, through the handpiece, reads the data from the component RFID. These data are transferred from the console to the surgical navigation system. Based on these data, the surgical navigation system processor generates data indicating the position of the component at the surgical site.

The above system is an efficient means for providing a surgical navigation system with information regarding the physical characteristics of surgical components. By repeatedly sending interrogation signals through the handpiece, the system is always provided with data identifying the specific component attached to the handpiece. However, this system requires a corded connection between the handpiece and a control console capable of reading the component RFID. For many components such as burs, shavers and saw blades, this is not drawback. These components are attached to and driven by powered handpieces. The power to such a handpiece is typically supplied through cable that extends from the control console. It is a simple task to build into the cable the conductors required for signal exchange with the component RFID.

Nevertheless, there are times where it is difficult to provide a corded connection to the instruments, implants and other components applied to a surgical site. For example, some surgeons employ battery-powered instruments. A surgeon uses this type of instrument because he/she does not want to introduce a cable into the vicinity of the surgical site. Some components, when applied to a surgical site, are not driven by powered handpieces. These surgical components include, implants, trial implants and hand powered tissue cutting and shaping tools. Attaching a cord to each of these components as part of surgical procedure to read their RFIDs can complicate and increase the time it takes to perform the procedure.

Several solutions have been proposed to the problem associated with providing component data to the surgical navigation system in the above situations. One solution is to provide a stand alone box that contains a coil for inductively exchanging signals with the component RFID. Prior to a new component being applied to the surgical site, the component is passed by the box. The RFID data are read by the box and forwarded to the surgical navigation unit. A second proposed solution involves providing a corded hand held pointer with a coil in its tip. Prior to the surgical component being applied to the surgical site, the operating room personnel place the pointer in close to the component RFID. The data in the component RFID are read by the pointer and transmitted to the surgical navigation system.

Both of the above solutions require extra components to be introduced into the operating room. To perform surgery as efficiently as possible, one tries to minimize the number of pieces of surgical equipment used to perform a procedure. Moreover, both solutions require the surgical personnel to perform an additional step, namely, to have the RFID of the new component read, as part of the procedure. Requiring the personnel to take this step increases the complexity of the procedure and overall time it takes for the procedure to be performed.

Moreover, in the event the steps are not taken to properly perform the identification procedure, the navigation system may provide incorrect information about the location or shape of the surgical component. This type of error may occur, for example, when a surgeon is using a number of different trial implants to determine the proper size or type of implant that should properly be fitted to the patient. Typically, a common handle is used to place the different implants at the surgical site. If, after the switching of the attached trial implant, the component identification step is not performed, the surgical navigation system may generate information that gives an inaccurate impression of the implant. Thus, it is desirable to automate as fully as possible the identification of components used in a procedure that are tracked by a surgical navigation system.

DE 102 39 673 A1 relates to compensating a muscle tremor and an unintended movement of a surgeon. An optical position recognition system detects suitable objects. These suitable objects are a drill and/or an equipment housing as well as a part of the body being operated. Actuators bring a tool independently of a movement of the surgeon and the patient to the desired position.

DE 102 41 070 A1 discloses a handle with an oscillating saw blade. The saw blade comprises a biochip embedded such that the surface of the biochip can get into contact with body fluids and body tissue. Such biochip can comprise coatings that respond selectively to certain DNA molecules. Within the saw blade electric wires are disposed, connecting the biochip with the interior of the handle, wherefrom the signals from the biochip are transmitted in an arbitrary way to a data processing means. These signals can be transmitted wirelessly. The handle may comprise a marker comprising reflecting bodies. These reflecting bodies reflect infrared radiation emitted from a navigation system for determining the position of the reflecting bodies.

US 6,434,507 B1 discloses an apparatus for use with a surgical navigation system. The apparatus comprises a tool body and tool attachment onto which emitters are fixedly mounted. At least one tool tip is removably coupled with the tool body. An electrical sensor is positioned to be operated when the tool tip is changed either by coupling the tip to or removing the tip from the tool body. A controller responsive to the operation of the switch detects when the tip has been changed. An alarm responsive to the controller indicates that the tool tip has been removed or changed so that the tip can be re-calibrated relative to a known position in the surgical navigation system.

US 2004/0092991 A1 discloses a surgical tool system with a hand piece and a cutting accessory that has inner and outer hubs. The handpiece has a spring bias lock ring that abuts a complementary stop member integral with the accessory outer hub to hold the accessory to the handpiece. An elastomeric seal pressed against the inner wall of the accessory outer hub provides environmental protection for the RFID chip. The seal has a rib that extends around the outer perimeter of the proximal end of the outer hub so as to form a seal between the accessory and an adjacent inner wall of the handpiece.

### SUMMARY AND INVENTION

The present invention provides a tracker according to claim 1.

This invention is related to a tracker for a surgical navigation system. said tracker and complementary components are integrated so that the tracker, upon attachment to a component, reads the data in a memory integral with the component. The data in the memory include information that describes the characteristics of the component. The tracker broadcasts the read data wirelessly to the static components of the system. The system, based on these data, the location of the tracker and the previously stored data, provides information that accurately describes the location of the component at the surgical site.

The tracker may provide information about components that, when applied to the surgical site, are static relative to the handles used to position them at the site. These components, sometimes referred to as implement include implants, bone anchors and trial implants. The tracker may also provide information about components that are moving when applied to a surgical site. These components include reamers and other surgical instruments used to remove and/or selectively shape tissue.

### BRIEF DESCRIPTION OF THE DRAWINGS

The invention is pointed out with particularity in the claims. The above and further objects, features and benefits of this invention are discussed in the detailed description below taken in conjunction with the accompanying drawings in which:

Figure 1 depicts a surgical navigation system

Figure 2 is a side view of a tracker base and the surgical components to which it is connected;

Figure 3 is an exploded view of the tracker base and components to which it is connected;

Figure 4 is an exploded view of the tracker base and handle prism and the components internal to these components;

Figure 5 is a top elevational view of a core;

Figure 6 is a top view of the handle of the handle assembly;

Figure 7 is a side view of the handle;

Figure 8 is a perspective view of the outer sleeve of the handle assembly;

Figure 9 is a side view of the bearing and coil fitted to the bearing;

Figure 10 is a perspective view of the bearing;

Figure 11 is a side view of the handle assembly shaft and the coil mounted to the shaft;

Figure 12 is a cross sectional view of the shaft taken along line 12-12 of Figure 11 wherein the components mounted internal to the shaft are shown in an exploded view;

Figure 13 is an exploded view illustrating the components integral with the reamer and the shaft head unit;

Figure 14 is a cross sectional and exploded view of the reamer and shaft head unit;

Figure 15 is schematic and block diagram of electrical components ;

Figure 16 is a diagram the different types of data stored in the memory of an RFID integral to a surgical component ;

Figure 17A, 17B and 17C collectively form a flow chart of the process steps executed;

Figure 18 is a perspective view of an positioner, sometime called an impactor,

Figure 19 is a cross sectional view of the positioner of Figure 18,

Figure 20 is a cross sectional exploded view of the positioner of Figure 18 of a acetabular shell that is fitted in place with the positioner;

Figure 21A and 21B collectively form a flow chart of the how the cup of Figure 18 is fitted with the positioner in the surgical navigation and reactive workflow process;

Figure 22 is a plan view of a broach handle;

Figure 23 is a cross sectional view of the broach handle of Figure 22;

Figure 24 is a perspective view of a stem inserter;

Figure 25 is an exploded view of the stem inserter of Figure 24;

Figure 26A is a plan view of how the stem inserter of Figure 24 is coupled to an asymmetric surgical implement, here an implantable femoral stem;

Figure 26B is a cross sectional view of the stem inserter of Figure 24;

Figure 26C is a perspective view of the alignment yoke of the stem inserter of Figure 24;

Figure 27 is a schematic of the electrical components of the stem inserter of Figure 24;

Figure 28 is a diagrammatic view of a surgical component, here an implant, to which plural RFIDs are mounted;

Figure 29 is a diagrammatic illustration of a bone in a fractured state;

Figure 30 is a diagrammatic illustration of a pin used to mark the position of a body component and the handle used to fit the pin; and

Figure 31 is a flow chart of the process steps executed to track the removal of body tissue and then return the tissue to the location from which the tissue was removed; and

Figure 32 is a depiction about how markers are presented on the display to facilitate the precise positioning of tissue

### DETAILED DESCRIPTION

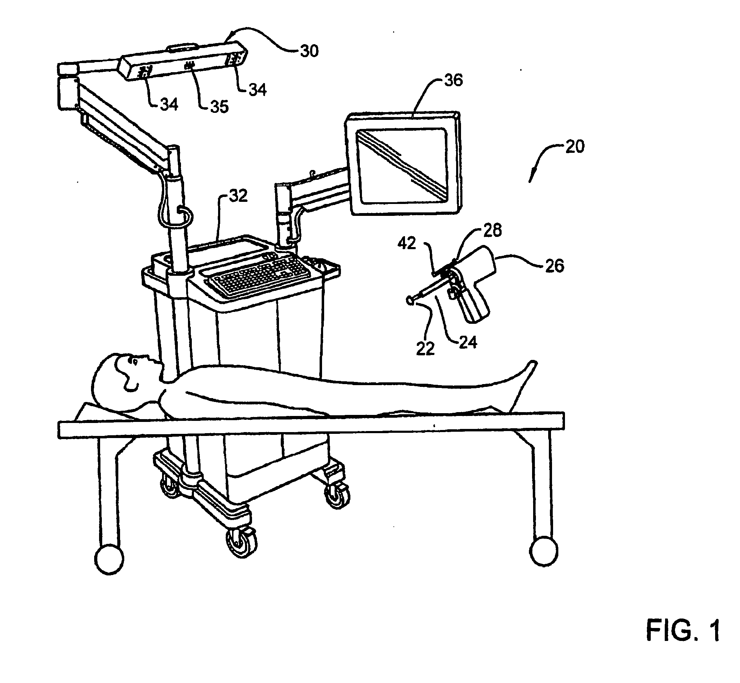

Figure 1 depicts a surgical navigation system 20 that obtains data about surgical components 22 and 24 without wire connections to the components. In Figure 1, surgical component 22 is a reamer. Surgical component 24 is a handle assembly. The proximal end of the handle assembly 24 is attached to a battery operated driver 26 that actuates the reamer 22. ("Proximal", it is understood, means away from the surgical site. "Distal" means towards the surgical site.) Not shown and not part of the invention is the battery attached to the base of the handgrip of the driver 26 that supplies the energization current for the driver. It should also be understood, and as will be discussed below "surgical component" may be any other instrument used to cut, form or shape tissue, a trial implant or an actual implant. Sometimes, the surgical component is alternatively referred to as a "surgical implement."

System 20 includes a tracker 28 attached to handle assembly 24. Tracker 28 broadcasts a specific form of energy from one or more emitters. Some trackers, for example, emit infra-red light or visible light. Other trackers emit RF energy, electromagnetic energy or sonic energy. Still other trackers emit sonic energy. A localizer 30, also part of system 20, monitors the position of the tracker 28. Specifically, the localizer 30 contains one or more receivers capable of receiving the energy emitted by the tracker 28. The receivers may determine the direction from which the energy is transmitted or the strength of the received energy.

The localizer receivers send signals representative of the measurements made thereby to a processor 32, also part of system 20. The processor based on the determination of the different locations from which the individual tracker transmitters emit energy or the strength of the received energy, the localizer 30 determines where, in three-dimension space, the tracker 28 is positioned and the orientation of the tracker. The data representative of the location and orientation of the tracker 28, or the signals used to determine this information, are forwarded by the localizer 30 to a processor 32 also part of the system 20. Processor 32, based on the signals from the localizer procedure, then generates signals representative of the position and orientation of the tracker 28. A more detailed explanation of how a surgical navigation system operates is contained in the Applicant's U.S. Patent Application No. 10/677,874, filed October 2, 2003, U.S. Patent Publication No. US 2004/0073279 A1.

As will be discussed in detail below, internal to the reamer 20 and handle assembly 24 are separate data storage devices. Each of these devices stores data that identifies the associated surgical component. These data include data that identify the physical characteristics of the component. When the reamer-handle-tracker sub-assembly is assembled, the tracker 28 reads the data in the storage devices. Tracker 28 transmits signals containing these data. A receiver 34, typically positioned in the localizer 30, reads these data. These data are transmitted by the receiver 34 to the system processor 32.

Prior to the commencement of the procedure in which system 20 is employed, the processor 32 is loaded with data that identifies the location of individual body tissues and organs at the surgical site. During the procedure, the processor 32 is provided with data indicating the location of the surgical site. Based on these data, the data indicating the position and orientation of the tracker 28 and the data identifying the physical characteristics of the surgical components 22 and 24, processor 32 generates information indicating the position of the surgical components relative to the surgical site. More particularly, the processor generates information indicating the position of the surgical components relative to the body tissue at the surgical site. Typically, this information is presented visually on a display 36. The surgeon is thus able to view the position of the surgical components that otherwise cannot readily be seen.

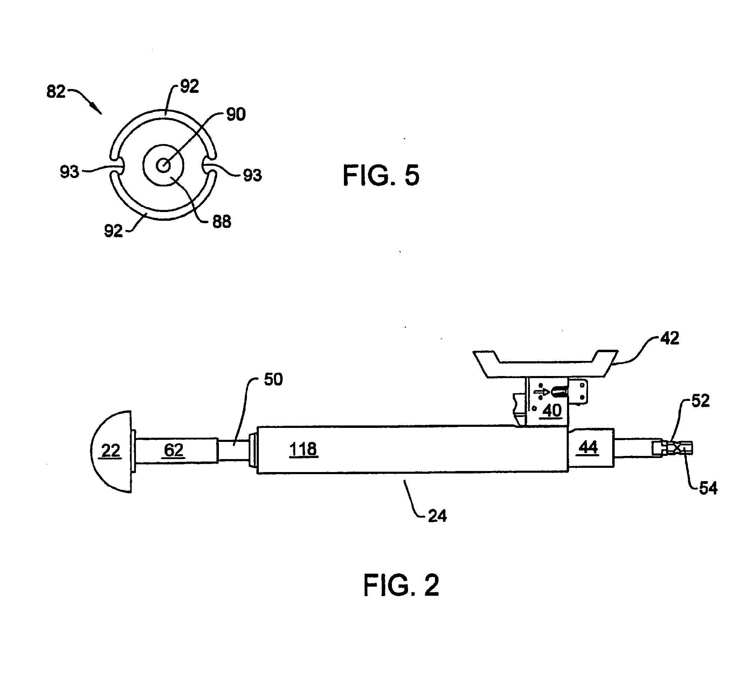

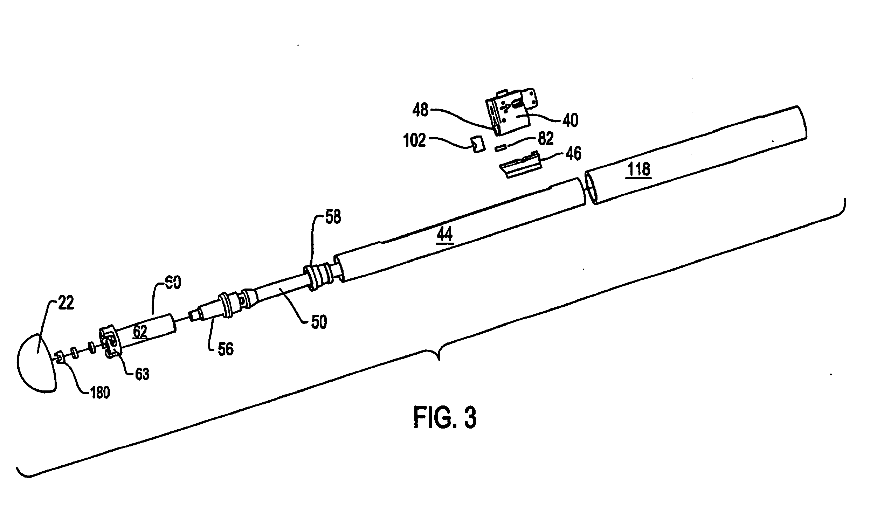

Figure 2 and 3 illustrate a tracker base 40 and the surgical components to which the base is connected, reamer 22 and handle assembly 24. The tracker base 40, is the bottommost component of the tracker 28. Located above base 40, tracker has a shell unit 42 (Figure 1). The shell unit 42 contains the components that transmit location and data signals to the static components of the system 20. Base 40 is fixedly attached to the shell unit 42 by an conventional fastening means such as threaded fasteners or welding.

The tracker base 40 is removably coupled to a generally tube-shaped handle 44 that is part of the handle assembly 24. A solid metal prism 46 is fixedly mounted to an outer surface of the handle 44 immediately forward of the proximal end of the handle. Tracker base 40 is formed to have a slot 48 along the bottom that extends the length of the base. Slot 48 is dimensioned to facilitate the tight slip fit of prism 46 into the slot. A locking mechanism, not shown; releasably tightly couples the tracker base 40 and prism 46 together.

A shaft 50, also part of handle assembly 24, is rotatably mounted in the handle 44. Shaft 50 has a proximal end tail 52 that extends rearwardly out of the proximal end of the handle 44. Shaft tail 52 is formed with geometric features 54 that facilitate the coupling of the shaft 52 to a complementary shaft internal to the driver 26. More particularly, the handle assembly shaft 50 engages the driver shaft so that the two shafts rotate in unison.

Shaft 50 also has a distal end 56 that is located forward of the distal end of the handle 44. A ring shaped bearing 58 is statically mounted in the open distal end of the handle 44. Bearing 58 functions as an interface between handle 44 and shaft 50.

A head unit 60 is attached to and extends forward from the shaft distal end 56. The head unit 60 contains a cylindrical stem 62 that is most proximal section of the head unit and is the portion of the head unit attached to the shaft 50, A crown 63, with a diameter wider than the stem 62, is formed integrally with stem 62 and is the most distal portion of the handle assembly 24. Crown 63 is formed with geometric features designed to couple the reamer 22 to the head unit 60. While not illustrated, it should be understood some type of locking device is attached to the head unit 60. The locking device, which is typically moveable along the length of the stem 62, includes locking member that, when selectively positioned, engage members integral with the reamer 22. These locking members releasably hold the reamer 22 to the handle assembly 24. One locking device is described in U.S. Patent No. 6,540,739 B2, SURGICAL INSTRUMENTATION SYSTEM, issued April 1, 2003.

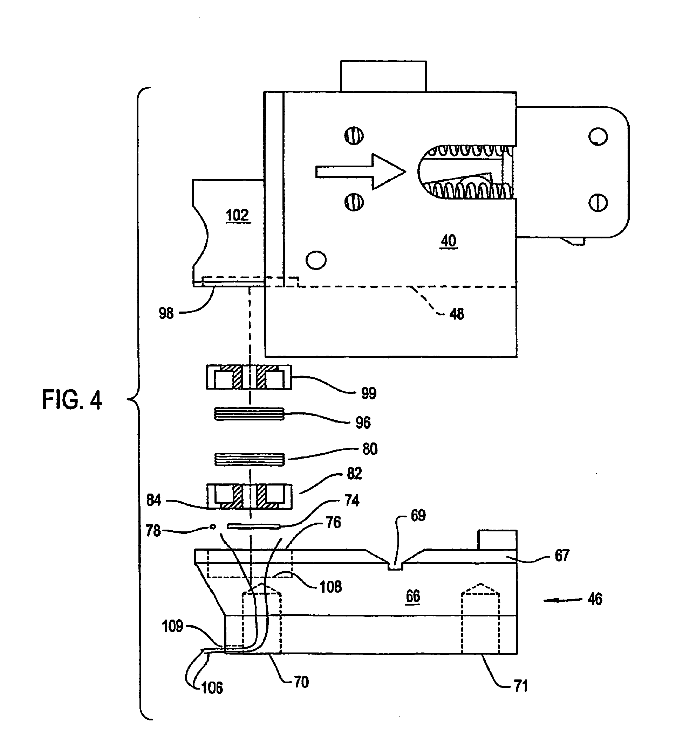

A more detailed description of the interface between the tracker base 40 and handle'prism 46 is now set forth by reference to Figures 3 and 4. Prism 46 is in form of a solid elongated block. The bottom portion, the base 64 of the prism 46, has a generally rectangular cross sectional profile. Integral with the base 64, prism 66 is formed to have a head 66 that tapers outwardly from the base. A crown 67, integral with and located on top of the head 66 forms the top most section of the prism 46. Prism 46 is further formed to have a notch 69 that extends laterally across the prism. The notch 69 cuts fully across the crown 67 and extends a short distance into the head.

Prism 46 is further formed with two longitudinally spaced apart bores 70 and 71, both shown in phantom. Bores 70 and 71 each extend upwardly through the prism base 64 are partially through the prism head 66. Bores 70 and 71 are dimensioned to receive positioning pins, not illustrated, that precisely hold the prism 46 to the handle 44. During the assembly process, the pins hold the prism 46 to the handle 44 so the two components, when welded together, are in a precise spatial relationship.

The tracker base 40 is formed so that slot 48 opens downwardly and is formed to extend forward from the proximal end of the base. (In Figure 4, the base of slot 48 is shown in phantom.) Slot 48 is dimensioned to allow the close slip fitting of the handle prism 46 into the slot. While not illustrated, it should be understood that internal to the tracker base is a moveable tongue positioned to tightly fit in prism notch 69. This tongue is part of the locking mechanism that holds the prism 46 to the tracker base 40. The tongue-and-groove fitting of prism 46 into slot 48 and of the tracker base tongue into prism notch 69 result in the tracker base 40 and prism 46 being tightly locked together.

An RFID 74 specific to handle 44 is mounted in the prism 46. The RFID 74 is seated in a downwardly extending bore 76 (shown in phantom) formed in the prism 46. The bore is located immediately rearward of the distal end of the prism 46 and extends through the prism crown 67 and a short distance into the prism head 66. The RFID 74 is located in the base of bore 76. Also located in base of the prism bore 76 is a capacitor 78. While not illustrated in Figure 4, the RFID 74 and capacitor 78 are connected together.

A coil 80 connected to RFID 74 and capacitor 78 is disposed in the prism bore 76 above the RFID and capacitor. The coil 80 is seated in a magnetic core 82 also seated in bore 76. The core 82 is formed from highly magnetic material such as ferrite. A ferrite core can be a ceramic formed from iron oxide mixed with an oxide or carbonate of manganese, zinc, nickel or magnesium. Core 82, as seen in Figures 4 and 5, includes a circular base 84. A boss 88 extends upwardly from the center of the base 84. The boss 88 is formed with an axially extending through hole 90. The core 82 is also formed with two arcuately shaped and spaced apart flanges 92. The flanges 92 extend upwardly from the perimeter of the base 84. Core 82 is also shaped so that in the interstitial gaps between the flanges 92, the base 84 is formed with notches 93

The coil 80 is seated in the annular space between the center located core boss 88 and the outwardly located flanges 92. The ends of the wire forming the coil 80, while not illustrated, extend downwardly through the base notches 93. A potting material (not illustrated) encapsulates the RFID 74, capacitor 78, coil 80 and core 82 in the prism bore 76. An epoxy may be used as the potting material. It should be understood that the potting material is able to withstand the rigors of autoclave sterilization.

Signals are inductively exchanged between the handle coil 80 and a coil 96 disposed in the tracker base 40. Specifically, the tracker base 40 is formed with a closed end bore 98 (shown in phantom) in which coil 96 is held. Bore 98 is formed in the tracker base 40 to extend upwardly from the distal end of the downwardly directed surface that defines the base of slot 48. In the depicted version, the tracker base 40 has a nose 102 that extends forward from the portion of the base that defines slot 48. The bottom surface of nose 102 is flush with the surface of the tracker base 40 that defines the tracker slot 48. The nose 102 is further formed so that a distal end section of the tracker base bore 98 is formed in the nose 102. Collectively, the tracker base 40 and prism 46 are formed so that, when the base is mounted to the prism, the tracker base bore 98 is in registration with the prism bore 76.

The tracker base coil 96 is seated in a core 99 similar to core 82. Potting material encapsulates the core 82 and coil 96 in the tracker base bore 98. Not illustrated is a bore formed through the tracker base that opens into the base of bore 98. This bore serves as the conduit wherein the wires to the coil 96 are connected to the other electrical components integral with the tracker 28. These components are disposed in the tracker shell unit 42.

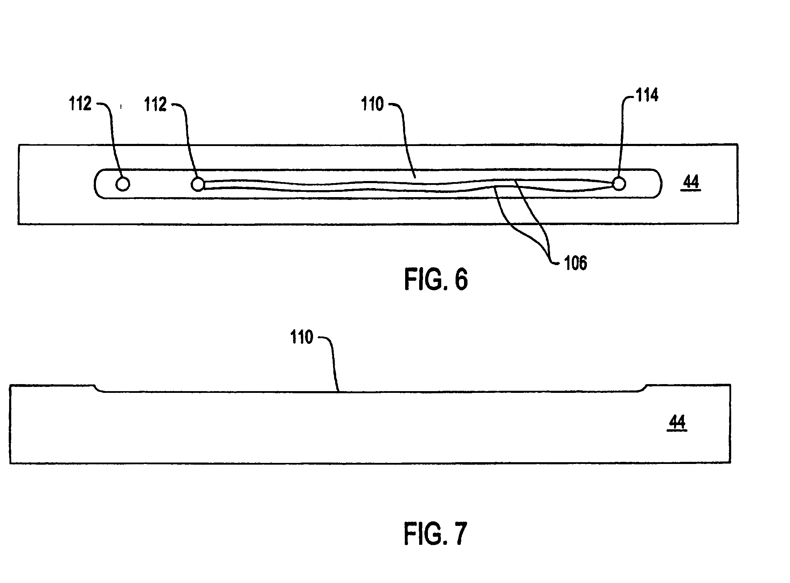

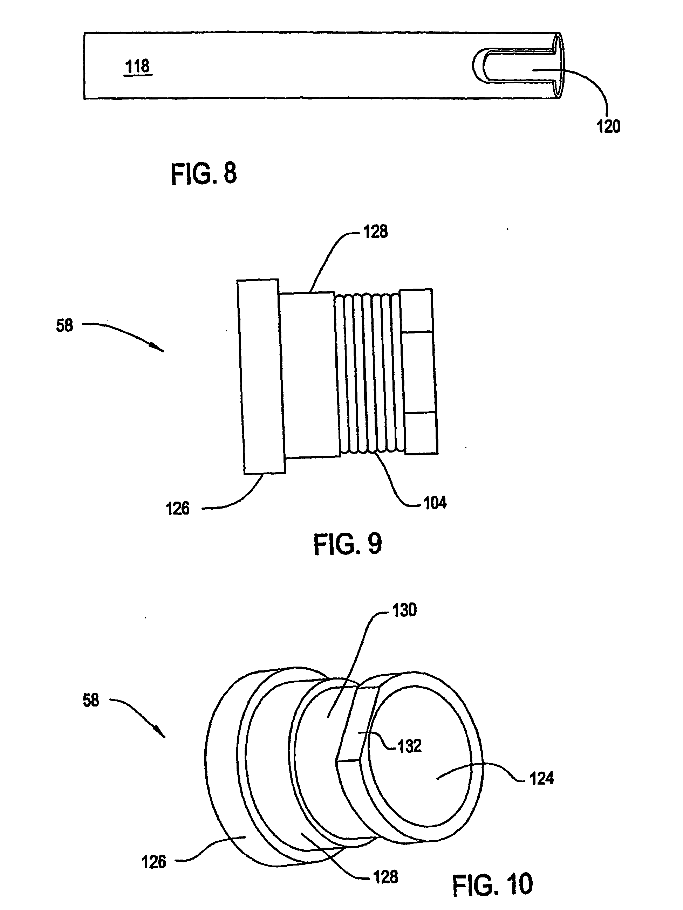

The handle coil 80 is also connected to a coil 104 disposed around the handle assembly bearing 58 (Figure 9). Conductors 106, seen best in Figures 4 and 6, connect coils 80 and 104 together. The conductors 106 extend from capacitor 78 through a through hole 108 (shown in phantom) formed in the base of prism bore 76. Hole 108 opens into the most distal of the bores, bore 70 that receives a positioning pin. The conductors 106 extend out of notch 109 (shown in phantom) formed in the bottom of the prism base 64. Notch 109 extends from bore 70 distally forward to the front end of the base 64.

The conductors 106 extend over an undercut face 110 formed on the outer surface of the handle 44 as depicted in Figures 6 and 7. Specifically, handle 44 is formed so that face 110 starts at a location forward of the proximal end of the handle and terminates at a location rearward of the distal end. Two through holes 112 are formed in the handle 44 to open into the proximal end of face 110. Through holes 112 receive the positioning pins that hold the prism 46 to the handle 44 during the fastening process. A third through hole, hole 114, extends from face 110 into the center of the handle 44 adjacent the distal end of the face. Through hole 114 functions as the opening through which conductors 106 are fed into the center void of the handle 44.

A generally tubular outer sleeve 118, now described by reference to Figures 3 and 8, extends over the handle 44. Sleeve 118 is open at both ends and is dimensioned to' closely slip fit over the handle 44. The sleeve 118 is formed with a slot 120 that extends forward from the proximal end of the sleeve 118. Slot 120 has a width equal to the width of the prism base 64. When the handle assemble 24 is assembled, the prism base 64 is seated in the sleeve slot 120. In the illustrated version, sleeve 118 extends from the distal end of handle 44 to the proximal end of prism 46. The handle 44 extends proximally a short distance beyond prism 46. Therefore, the proximal end of the handle 44 is not encased within the sleeve 118.

Bearing 58, now described by reference to Figures 9 and 10, is formed from a single piece of low friction material such as PEEK plastic. The bearing 58, as seen by reference to Figures 9 and 10, is formed to have an axially extending through bore 124. Bore 124 is dimensioned to allow the shaft 50 rotate freely in the bore. The bearing 58 is shaped on the outside to have a circular head 126. Bearing head 126 has an outer diameter equal to the outer diameter of handle 44. Extending proximally from the head 126, the bearing 58 has a stem 128. Stem 128 generally has a diameter that allows the close slip fit of the stem into the distal end opening of the handle 44.

The bearing 58 is further formed to have an inwardly directed undercut 130. Specifically, the undercut extends circularly around the stem 128 and is located forward of the proximal end of the stem. Coil 104 is disposed in the annular space located above undercut 130. The proximal end of.the stem 128, the end rearward of the undercut 130, is formed to have a planar face 132. Face 132 extends across an arcuate section of the stem 128. Undercut 130 defines a space between the inner surface of handle 44 and bearing 58 through which conductors 106 extended to coil 104.

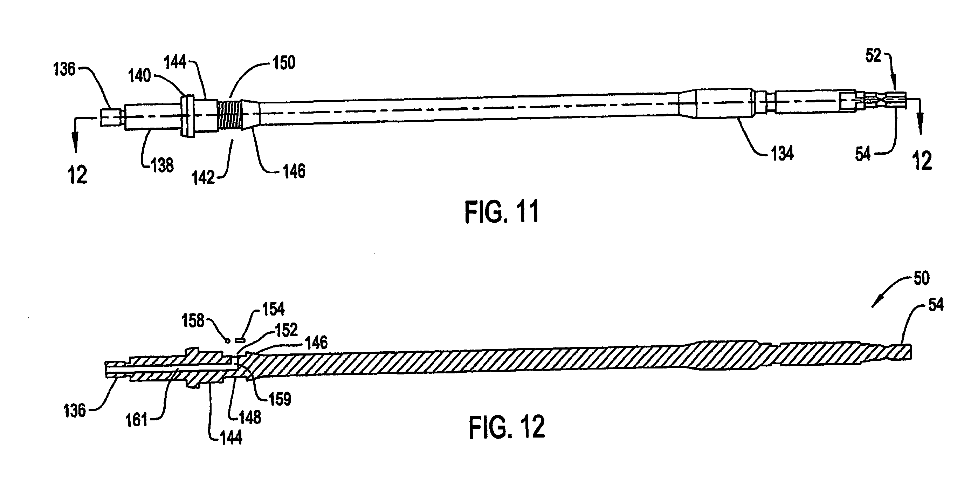

Handle assembly shaft 50 is now described by reference to Figures 11 and 12. The shaft 50 is the form of a generally rigid rod. The shaft 50 includes the proximal end tail 52 with features 54 that facilitate the coupling of the tail to a shaft assembly integral with the driver 26 so that two shafts rotate in unison. Forward of tail 52, the shaft 50 has a trunk section 134 with a diameter slightly larger than the diameter of the body of the shaft. A bearing, not illustrated, is disposed in the proximal end of the handle 44. The bearing extends to the trunk section 134 to provide a second low friction interface between the handle 44 and the shaft 50.

At distal end 56, shaft 50 is formed to have a head 136 that has an outer diameter less than that of the main body of the shaft. Extending proximally from the head 136, the shaft 50 has a neck 138 with a diameter equal to that of the main body of the shaft. The neck 138 extends from a collar 140. The collar 140 has a diameter greater than that of main body of the shaft. More particularly, collar 140 has an outer diameter greater than the diameter of bearing through bore 124. Thus, when handle assembly 24 is assembled, the shaft 50 is positioned so that collar 140 is located immediately forward of the bearing head 126.

Located immediately proximally rearward from collar 140, shaft 50 has a shoulder 142. The shoulder 142 has a distal section 144 with a constant diameter outer surface. The outer diameter of the shoulder distal section 144 is selected to allow a close rotating fit between the shoulder and the adjacent inner surface bore-defining wall of the bearing 58. Shoulder 142 has a proximal section 146 that is longitudinally spaced from the distal section 144. The shoulder proximal section 146 is shaped so as to taper outwardly along the length of the section 146 such that the diameter increases from the shaft main body towards the shaft head 136. The most distal portion of the shoulder proximal section 146 has a diameter equal to that of the distal section 144. Between the shaft distal and proximal sections 144 and 146, respectively, there is an intermediate section 148. Intermediate section 148 has an outer diameter substantially equal to that of the main body of the shaft 50.

A coil 150 is wound in the annular space immediately above the shoulder intermediate section 148 and between the distal and proximal sections 146 and 148, respectively. Coil 150 is wound over a counterbore 152 that extends laterally towards the longitudinal center axis of the shaft from the shoulder intermediate section. An RFID 156 that contains data specific to the shaft 50 is disposed in the counterbore 152 below coil 150. A capacitor 158, also connected to the coil 150, is also located in the counterbore 152.

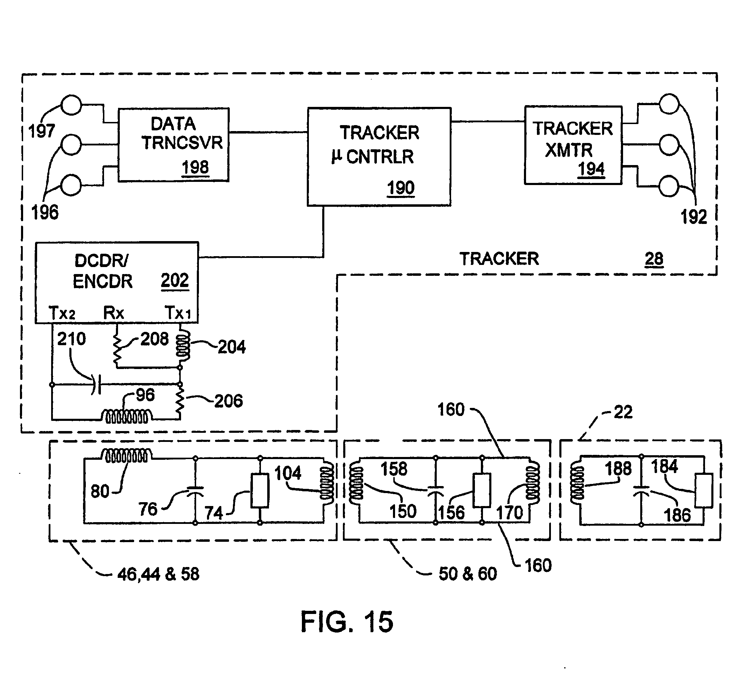

Conductors 160, shown schematically in Figure 15, extend forward from the coil 150, the RFID 156 and capacitor 156 through the shaft 50. Specifically, the shaft 50 is formed with a bore 159 that opens from the base of counterbore 152 into the longitudinal center axis of the shaft 50. Shaft 50 also has a bore 161 that extends from the shaft head 136 proximal to bore 162. Conductors 160 extend through bores 159 and 161. Potting compound encapsulates coil 150 over shaft shoulder intermediate and RFID 156 and capacitor 158 in counterbore 152.

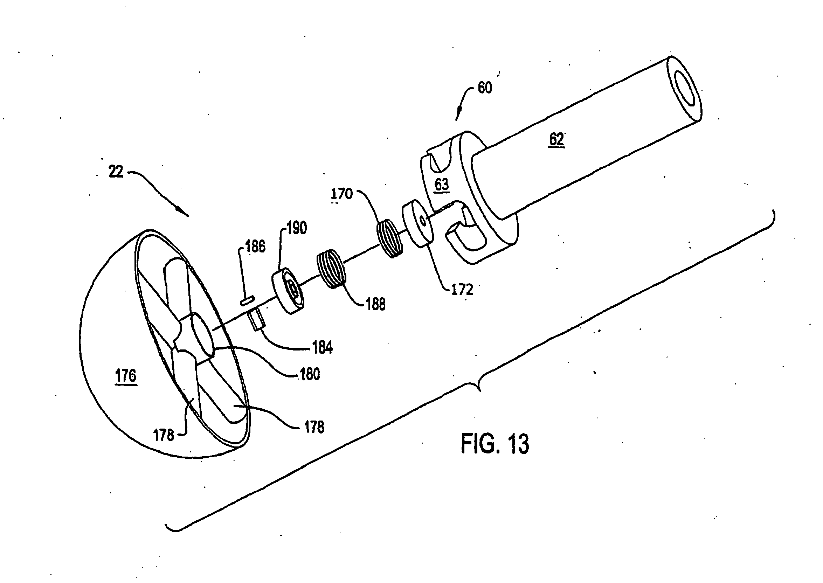

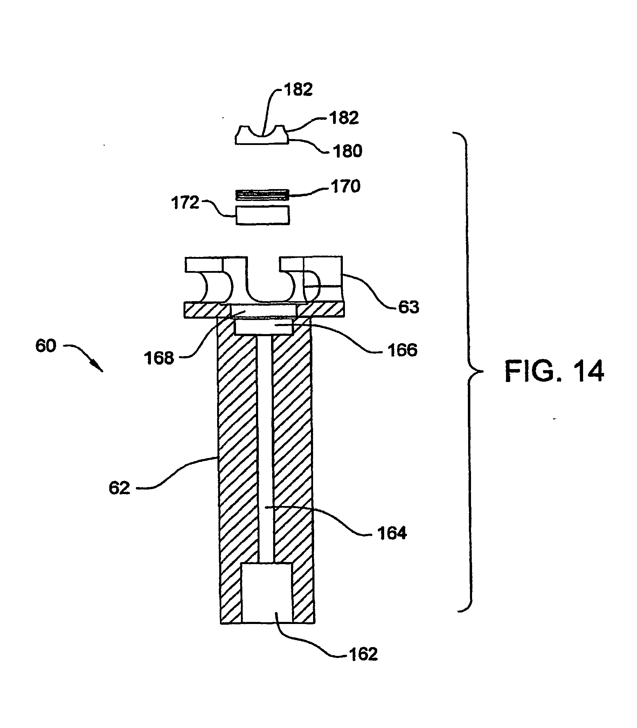

The shaft head unit 60, now described by reference to Figures 13 and 14, is secured to the shaft head 136. Specifically, the head unit 60 is formed with a threaded bore 162 that extends forward from the unit stem 62. The shaft head 136 is formed with complementary threading to facilitate the attachment of the head unit 60 to the shaft 50. (Bore and head threading not illustrated.)

Shaft head unit 60 is further formed to have a bore 164 that extends axially through the unit stem 62 from the base of bore 162 towards the unit crown 63. Bore 164 opens into first and second counterbores 166 and 168. The first counterbore 166, the bore into which bore 164 opens, is located in the unit stem 62. The second, outer counterbore, counterbore 168, is formed in a circular plate that is part of the unit crown 63. Second counterbore 168 has a diameter greater than that of the first counterbore 166.

Conductors 160 are connected to a coil 170 seated in the first counterbore 166 of the shaft head unit 60. Coil 170 is seated in a core 172 similar to core 82. Potting compound encapsulates the coil 170 and core 172 in the first counterbore 166. The second counterbore 168 is open to the environment.

Reamer 22 includes head 176. In the depicted version, head 176 is semi-spherical. It is appreciated by those familiar with the design of reamers that this shape is exemplary, not limiting. The head 176 is formed with cutting surfaces. In Figure 13, a plurality of scalloped edges 177 represent the cutting surfaces. The exact geometry of the cutting surfaces is not relevant to this invention. Two perpendicularly aligned cross bars 178 extend across the inner surfaces of the head. The cross bars 178 are the reamer structural members that engage the components of crown 63 of the shaft head unit 60.

The reamer 22 also has a cup 180 that is welded or otherwise secured to the cross bars 178 at the points the cross bars intersect. Cup 180 is generally ring shaped. The distal end of the cup 180 is formed with four equiangularly spaced apart concave surfaces 182. When cup 180 is fitted to the reamer 22, cross bars 178 seat in the circular spaces defined by surfaces 182. Typically, the cup 182 is laser welded to the cross bars 178.

An RFID 184 that contains data specific to the reamer 22 is disposed in the center space internal to the cap 180. A capacitor 186 and coil 188 are connected in parallel across the RFID 184. The coil 188 is seated in a proximally-directed core 190 disposed in the cup 180. The RFID 184 and capacitor 186 are seated in the cup 180 between the cross bars 178 and the base of the core 190. Potting compound encapsulates the RFID 184, the capacitor 186 the coil 188 and the core 190 in the cup 180.

Cup 180 is shaped to have an outer diameter that is slightly less than the diameter of the shaft head unit second counterbore 168. The cup 180 is dimensioned so that, when the reamer 22 is coupled to the shaft head unit 60, the proximal end of the cup 180, the end containing coil 188, seats in the head unit second counterbore 168.

Figure 15 is a block and schematic drawing of the electrical components integral with the reamer 22, the handle 24 and tracker 28. The tracker 28 has a primary microcontroller 190. Microcontroller 190 regulates the overall operation of the electrical components internal to the tracker 28. One suitable unit that can be employed as the microcontroller 190 is from the C51 family manufactured by Philips Semiconductor. Integral with the microcontroller 190, though not illustrated, is a memory that stores program instructions executed by the microcontroller and data received by and transmitted from the tracker 28.

The tracker 28 includes a set of one or more energy transmitting devices that transmit signals used to identify the location of the tracker. In Figure 15 these devices are infra-red light transmitting LEDs 192. The actuation of the LEDs 192 is regulated by a tracker transmitter 194. The actual pattern in which the LEDs 192 are switched on and off is regulated by command signals generated by microcontroller 190.

Tracker 28 also contains a set of devices that exchange data signals with the static components of the surgical navigation system 20. In the tracker of Figure 15, these devices are a second set of infra red light transmitting LEDs 196 and a photosensor 197. In Figure 1 a rectangular phantom box 35 in the localizer represents the complementary transceiver.

The on/off sequences of the individu'al LEDs 196 are set by a data transceiver 198. Data transceiver 198 causes the LEDs 196 to be actuated in order to send a specific data stream based on instructions from the microcontroller 190. Transceiver 198 also receives signals generated by photosensor 197 in response to the receipt of infra-signals generated by a transmitter integral, (not illustrated). In response to the signals generated by photosensor 197, data transceiver 198 generates data containing signals that can be read by microcontroller 190.

It should be understood that in alternative versions, transceiver 198 is capable of exchanging RF signals with the complementary external transceiver 35. In these versions of the invention LEDs 196 and photosensor 197 are replaced by an antenna, (not illustrated).

The tracker coil 96 is connected to a decoder/ encoder 202 also internal to the tracker. One suitable decoder/encoder is the HTC110 manufactured by Philips Semiconductor. More particularly, the Tx₁ pin of the decoder/encoder 202 is connected to through a series connected inductor 204 and resistor 206 to one end of coil 96. The Tx₂. pin of the decoder/encoder 202 is directly connected to the opposed end of coil 96. A resistor 208 extends from the Rx pin of encoder/decoder 202 to the junction between the inductor 204 and resistor 206. A capacitor 210 extends from the inductor 204-resistor 206 junction to the Tx₂ pin.

The above circuit is constructed so that each inductor-capacitor sub-circuit is tuned to a specific and identical resonant frequency. The specific frequency is the frequency at which the RFIDs 74, 156 and 186 exchange signals. In one particular version, this frequency is 125 K Hz. Thus, inductor 204 and capacitor 210 are tuned to this frequency. Coils 80 and 104 and capacitor 76 of the prism-handle-bearing sub-assembly are similarly tuned to this frequency. Coils 150 and 170 and capacitor 158 of the shaft 50 and head unit 60 are tuned to this frequency. The circuit integral with the reamer 22, capacitor 186 and coil 188 are likewise tuned to this frequency.

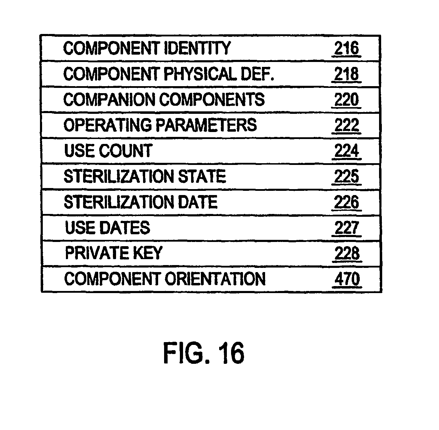

Each RFID 74, 156, 184 has a memory in which data describing the surgical component or the surgical component subsassembly with which the RFID is integral are stored. The RFID 74 integral with the handle, as represented by the data fields depicted in Figure 16, stores data identifying the type of handle, component identity field 216. These data may be alphanumeric data. If the data are in this form, the data is simply presented in the appropriate location on the display 36. Alternatively, these data may be a code. The memory integral with the system processor 32 contains a look up table with these codes and data indicating which code corresponds to a particular instrument. The component identity data stored in the RFID 74 also includes serial number, manufacturer, manufacturing lot number and manufacturing date data. While not shown in Figure 16, it should be appreciated these data are separate data fields.

The memory internal to RFID 74 also has data describing the physical characteristics of the prism-handle-bearing sub-assembly, represented by physical definition field 218. These data describe the physical structure of the sub-assembly. These data, for example, may include the length and diameter of the sub-assembly. In surgical components having complex geometry, for example implants and trial implants, there may be significant amounts of data that collectively describe the physical structure of the component.

The memory of the RFID 74 also includes data indicating the components with which the handle sub-assembly can be used, companion component field 220. For example, there may only be certain shafts that should be fitted to the handle-subassembly. In this situation, companion component field 220 contains data that identifies the specific shafts that can be used with the handle assembly.

RFIDs 156 and 184 integral with, respectively, shaft 50 and reamer 22, contain data similar to that within RFID 74. RFIDs 156 and 184, also contain, in operating parameters fields 222, data that describes how these components should be actuated. These data include a general preferred and maximum operating speed at which these components should be actuated and a maximum torque the component should be allowed to develop.

Each RFID 74, 156 and 158 also contains one or more data fields to which the tracker 28 is able to both read and write data. These fields include a use count field 224. Field 224 is used to store data indicating the number of times the particular component is used. There is a sterilization state field 225. Field 225 contains data indicating whether or not a particular component has been sterilized. Typically, after a component has been sterilized, a data decoder/encoder at the sterilization facility sets the data in field 225 to indicate that the component has been sterilized. There is a sterilization date field 226. The sterilization facility data decoder/encoder is used to write into field 226 the data the component was last sterilized. The RFIDs also include a use history field 227. Each time the component is used, the tracker writes the data of use into field 227. Thus, field 227 contains a record of the dates the component was used. Field 227 in some versions of the invention contains one or more subfields in which data identify the surgeon using the component, the name of the patient on which the component was used or the type of procedure in which the component is used are stored.

While not shown, the RFID memory also contains fields indicating one or more of the following types of data: the total amount of time the component has been used; and the amount of time of use remaining before the component should be subjected to a maintenance overall. The use data are written to the component RFID by the tracker 28 as a consequence of the use of the component. The use remaining data are initially written into the memory at the maintenance facility. The data are then updated by the system processor 32 and tracker 28 as a result of the use of the component.

. In some versions, one or more of the above types of data may be encoded. The data may be encoded under a public key. The RFID 74, 156, and 184 each contains, in a private key field 228, a private key to decode the companion data. The private key field 228 is one of the data fields in the RFID that can be overwritten. Thus, after the associated component has reached its use limit, the tracker 28 overwrites the data in the private key field 228. This makes it impossible to decode the encoded data.

While not illustrated, it should also be recognized that the RFID memory contains fields with error correction data.

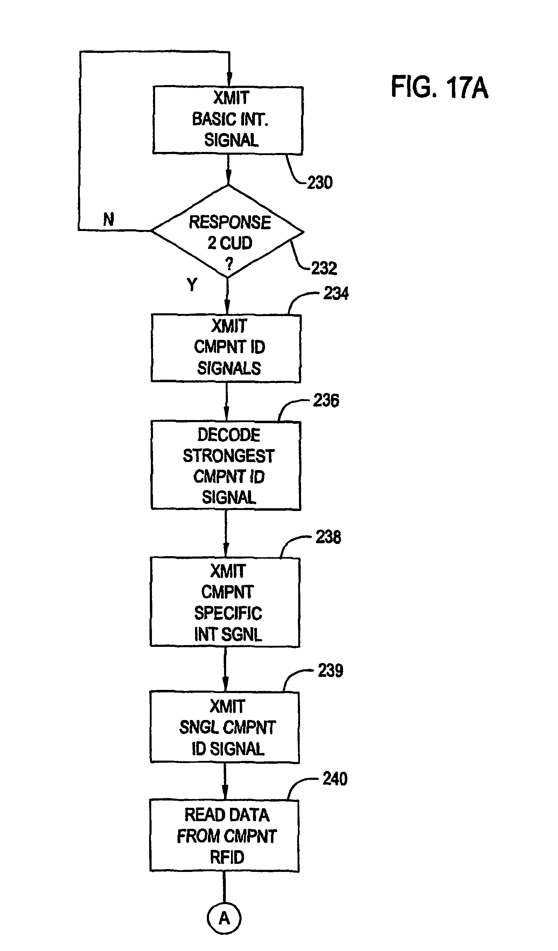

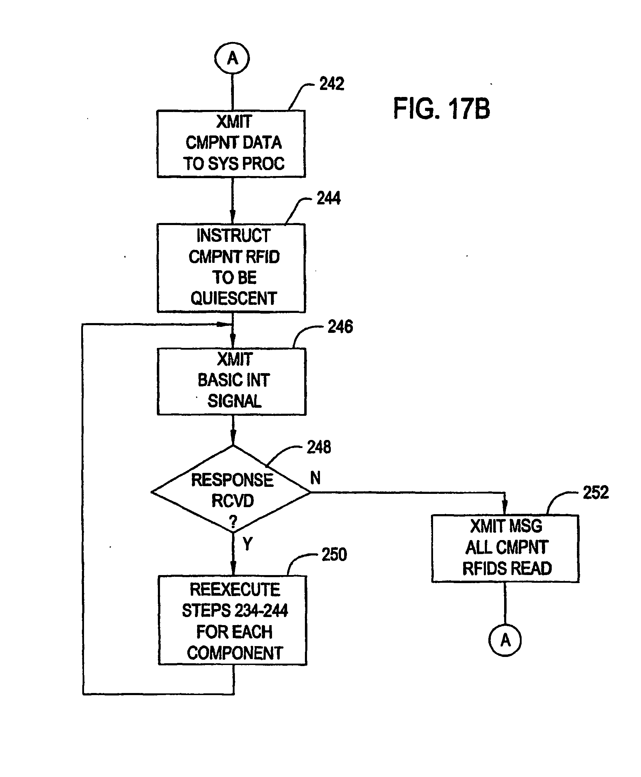

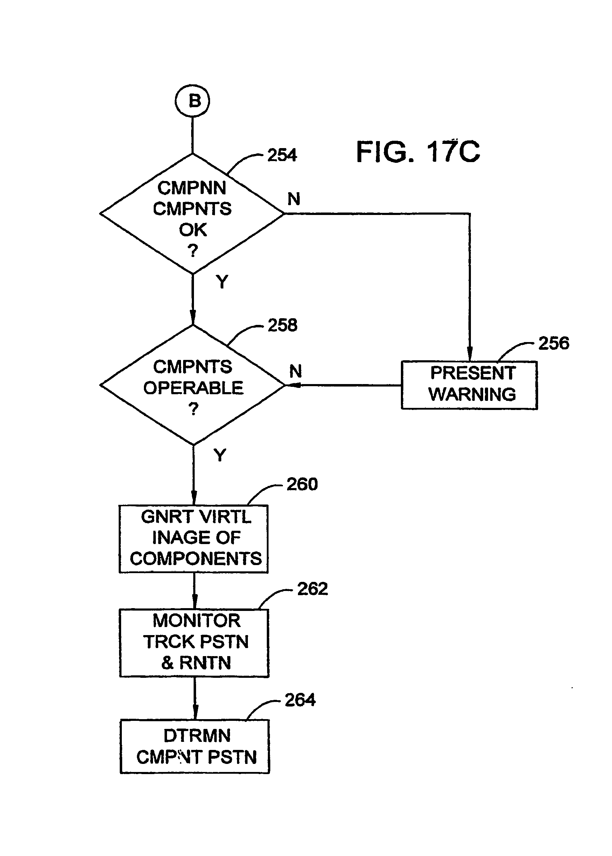

System 20 is prepared for use by fitting reamer 22 to the handle assembly 24, attaching the handle assembly to the power driver 26 and attaching the tracker 28 to handle assembly. Once the tracker 28 is actuated, the tracker microcontroller 190 continually instructs the decoder/encoder 202 to send out a interrogation signal through coil 96, step 230 in Figure 17.

It should be appreciated that the decoder/encoder 202 generates output signals in the form of square waves. Inductor 204 and capacitor 210, not coil 96, convert the square wave signals into sinusoidal signals for the inductive signal transfer. Since coil 96 is not part of the sub-circuit that generates the sinusoidal signals, changes in the load downline from the tracker 28 neither results in significant attenuation nor significant phase shift in the signals applied across the coil 96.

If, in response to a basic interrogation signal, the tracker 28 does not receive a response, the microcontroller 190 recognizes the tracker 28 as being in a state wherein no surgical component is attached, step 232. The tracker then continues to reexcute step 230.

When the reamer and handle assembly is attached to the tracker 28, in response to the basic interrogation signal, each RFID 74, 156 and 184 initially, and near simultaneously, respond by sending back a component identification signal, step 234. This signal based on data stored in the component identity field 216 and provides a basic identification to the tracker of the component.

The tracker decoder/encoder 202 only reads and decodes the strongest of these signals, step 236. Typically, this is the signal of the closest RFID. In the described configuration, this is the RFID 74 integral with the handle 44. As part of step 236, the decoded identification data are sent to the tracker microcontroller 190.

In response to a receipt of this specific basic identification, the tracker microcontroller 190 and the decoder/encoder 202, in step 238, generate a component specific integration signal to the handle RFID 74 directing it to affirmatively acknowledge its present. The handle RFID 74 responds to this inquiry in step 239.

As a result of the handle RFID 74 acknowledging its presence, the tracker 28, in step 240, reads out the data from the handle RFID 74. Once these data are read, the microcontroller 190, in step 242 transmits the data through transmitter 198 and LEDs 196 to the static receiver 34. Once the data read is complete, in step 244, the tracker microcontroller 190, instructs the handle RFID 74 to not respond to the next basic interrogation query.

Tracker 28 again performs a basic interrogation step, step 246. Given the prior instructions received by the handle RFID 74, in response to this inquiry, the RFID. 74 does not respond. Instead only the shaft RFID 156 and reamer RFID 184 respond, step 248. Since the shaft RFID 156 signal is the stronger of the two signals, this is the RFID that is next read by the tracker 28. In other words, steps 236-244 are reexecuted. Only during this sequence of the execution of these steps, the data from the shaft RFID are read. These steps are shown collectively as step 250.

A third basic interrogation signal is then generated. In other words, step 246 is reexecuted. At this time, both the handle RFID 74 and shaft RFID 170 are instructed not to respond. Therefore, in response to the second execution of step 246, only the reamer RFID 184 responds. Then, in the second reexecution of steps 236-244, the data stored in the reamer RFID 184 are read and forwarded to processor 32.

Basic interrogation step 246, is then reexecuted. At this time, each of the RFIDs 74, 156 and 184 is operating under an instruction not to respond. Therefore, since no response is received to the interrogation, in step 248 the tracker microprocessor 190 recognizes the system as being in a state in which the data stored in each component RFID has been read. The microprocessor 190 causes a message informing the system processor 32 of this fact in step 252.

Based on the data received from the RFIDs 74, 170 and 184, the system processor 32 first determines whether or not the system is appropriately configured. For example, the system determines whether or not the reamer 22 is appropriate for the shaft 60. This step is performed by reference to the companion component data from field 220 for the shaft 60 and the component identity data from field 216 for the reamer 22. If the data indicate that the reamer is not appropriate, the processor 32 causes an appropriate warning to be presented on the display 36, steps 254 and 256.

System processor 32, in step 258, also determines whether or not the components are actually useable. This step is determined by making sure, based on data in the use count fields 224, sterilization state fields 225 and sterilization data fields 226, the useful life time of each component has not been exceeded, the component is sterilized, based on the date of sterilization, can still be considered sterilized, and the component is not in need of service. If there is an indication the component cannot be used, an appropriate warning message is presented, step 256 is executed

If the surgical components are appropriately assembled, the processor 32 assembles a virtual image of the surgical component assembly, step 260. This image is based on the received data describing the physical characteristics of the components from the physical definition fields 218.

During the actual performance of the procedure, the system monitors the position of the tracker 28, step 262. Based on the position of the tracker 28, the stored data describing the position of the surgical site and adjacent body tissue and the virtual image of the surgical components, processor 32, in step 264, generates information indicating the position of the surgical components relative to the surrounding site and body tissue. Typically, this information is presented visually on display 36.

Throughout the time the tracker 28 is actuated, microcontroller 190 repetitively causes interrogation signals to be resent. In other words, basic interrogation signals are sent to cause a series of Component specific interrogation signals to be sent. If, in response to the signals, the same component identification data are received, microcontroller 190 recognizes the assembly as being one in which no components have been switched.

However, if, for example, a different sized reamer 22 is attached, the received component identification data will be different. Based on the receipt of these data, the tracker reads out all of the data from the RFID 184 of the new reamer. Based on these data, the processor 32 generates a new virtual image that reflects the size and shape of the new reamer.

System 20 continually generates information indicating the location of the surgical components 22 and 24 relative to the surgical site. Should, during the surgical procedure, the components change, the system 20 remains able to generate this information. System 20 does this by without requiring a wired connection between the components and the static equipment. Thus, the system 20 does require the use of corded equipment in order to obtain data that describes the new surgical components that are being introduced into the surgical site.

Moreover, system 20 automatically reads the data from the new surgical components as soon as they are attached to the tracker 28. Surgical personnel are not required to take any active steps to ensure that the data describing these components are read. The possibility that system 20 will generate information based on the assumption that a removed component is still in use is essentially eliminated.

Another feature of system 20 is that the coils 80, 96, 104, 150, 170 and 188 that perform the inductive signal transfer are not the sole components responsible for generating the sinusoidal signals that are so exchanged. In the tracker 28, this function is performed by capacitor 210 and inductor 204. In the downline components, this function is performed by the coils and associated capacitors. A benefit of this arrangement is that a change in circuit load as a result of the change of the downline components does not cause the significant attenuation or phase shift of the signals from those components.

System 20 of this invention also has a means to prevent the unauthorized reading of the data in the RFIDs. Specifically, if it is determined that the data in an RFID for a particular component should not be read, the tracker 28 only has to destroy the data in the private key field 228 for the RFID. This prevents the decryption software in the tracker 28 or processor 32 from being able to decode the encrypted data.

It should be recognized that the system 20 can be used to provide data about surgical components other than those that are actively driven such as reamers and other cutting devices. System 20 provides information about other surgical implements such as manually actuated tools such as rasps. Still other surgical implements system can be used to provide information about include trial implants and implants themselves. A manually actuated tool may be provided with an RFID that is attached to the mounting member to which the associated tracker 28 is attached. The instrument data are read from the tracker 28 and broadcast by the tracker to the system processor 32.

If the tool has moving parts, a sensor or sensors monitor the positions of these parts. For example, a potentiometer is used to monitor the extent to which the arms of forceps are open and closed. The signal across the potentiometer is digitized by a circuit internal to the forceps and transmitted to the tracker over the coils used to perform the inductive data transfer. The tracker then broadcasts a signal containing the data indicating the open/closed position of the forceps arms. In this way, the system processor 32 generates a near instantaneous virtual image of the forceps in their current open/closed state.

Typically, a handle is used to fit an implant or a trial implant to a patient. An RFID and coil are contained inside the implant or trial implant. Information similar to that carried in the reamer RFID 184 are stored in these components.

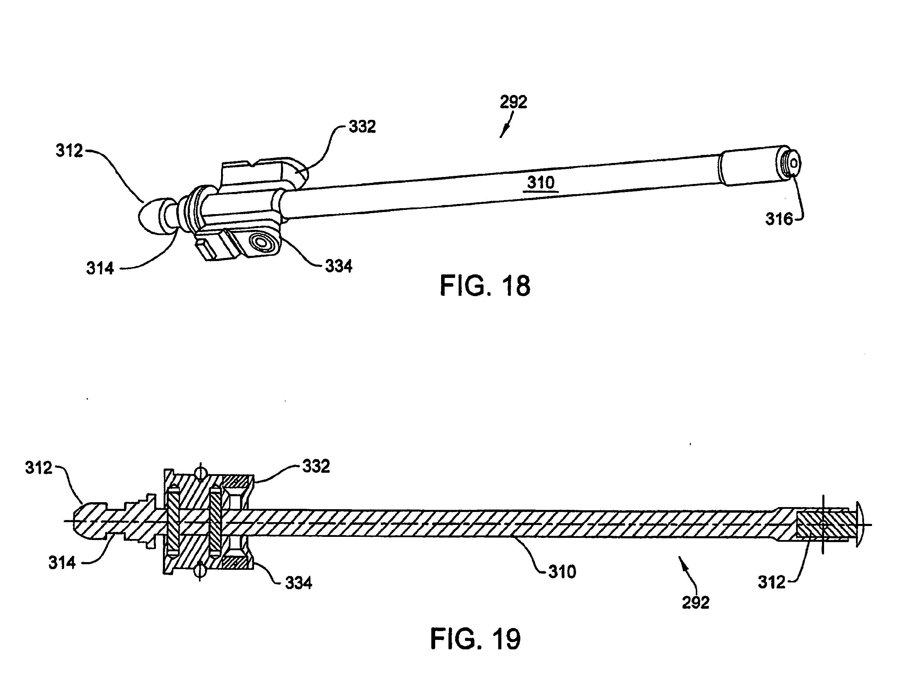

One such embodiment of the above described version is now described by reference to Figures 18-20. These Figures illustrate an acetabular shell 290 and the positioner 292, sometimes called an impactor, used to fit the shell. Shell 290 is the outer part of an artificial acetabular cup that is fit to the hip bone as part of the joint replacement process. Positioner 292 is a manually actuated tool used to drive, compression fit, shell 290 into the bone.

Shell 290 has a generally has a generally semi-spherical shape and is hollow. Shown as outwardly extending spikes 296 are surface features that facilitate the locking of the shell 290 to the bone. An open ended sleeve 298 extends outwardly from the inner surface of the shell 290. Sleeve 298 is centered along the longitudinal axis of the shell. The inner wall of sleeve 298 is provided with threading to facilitate the releaseable coupling of the shell 290 to positioner 292.

An RFID 302 is disposed in the base of sleeve 298. RFID 302 stores data similar to that in reamer RFID 184 (Figure 13). A coil 304 is connected to and disposed above RFID 302 in the sleeve 298. An encapsulating material 306 both holds the RFID 302 and coil 304 in position and protects these components from boy fluids.

Positioner 292 has an elongated shaft 310. At the proximal end, positioner 292 is formed with a rounded knob 312. A stem 314 having a diameter less than that of shaft 310 and the adjacent section of the knob 312 holds the knob away from the adjacent proximal end of the shaft. Collectively the knob 312, the stem 314 and the adjacent proximal end of the shaft 310 are dimensioned to removably receive a grip, sometimes called a handle, (not illustrated). The surgeon strikes the grip with a mallet so that positioner 292 drives the attached shell 290 into the bone.

A cylindrical boss 316 extends forward from the distal end of the positioner shaft 310. The outer circumferential surface of boss 316 is formed with threading, (not identified) dimensioned to engage the complementary threading integral with shell sleeve 298. Positioner boss 316 is also formed to have closed end bore 318 that opens proximally rearward from the front face of the boss. A coil 320 is disposed in bore 318. Coil 320 is positioned so that when the positioner boss 316 is screw secured in shaft sleeve 298 inductive signal exchange between coils 304 and 320 is possible. An encapsulating material 322 capable of withstanding the rigors of autoclave sterilization holds coil 320 in bore 318.

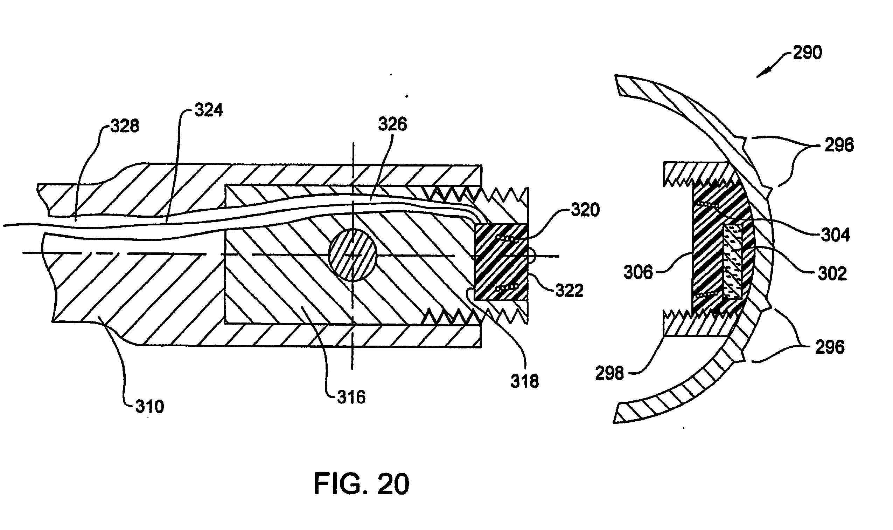

Conductors 324, shown as a single line in Figure 20, extend proximally from the opposed ends of the coil 320. The conductors 324 are disposed in a bore 326 that leads proximally from the base of boss bore 318 and into a bore 328 that extends through shaft 310.

### (Bores 326 and 328 seen best in Figure 20.)

Two prisms 332 and 334 are attached to the proximal end of positioner shaft 310. Prisms 332 and 334 are each like the reamer handle prism 46 (Figure 4.) Thus each prism 332 and 334 includes both an RFID that contains data describing the positioner 292 and a coil for inductively exchanging signals with the complementary tracker coil 96 (Figure 4). The RFIDs integral with each prism 332 and 334 also contain data fields in which data identifying the location at which the associated prism is attached to the positioner shaft 310. Conductors 324 that extend from coil 320 are connected to the RFIDs and coils of both prisms 332 and 334.

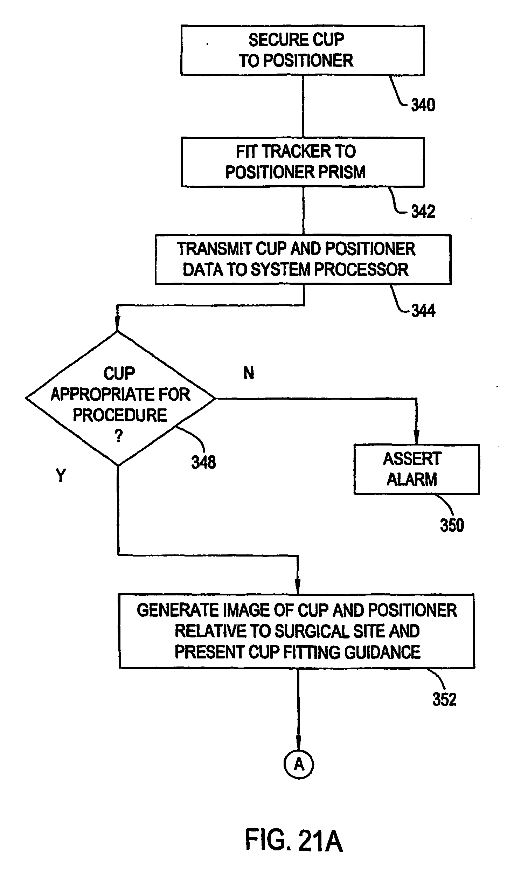

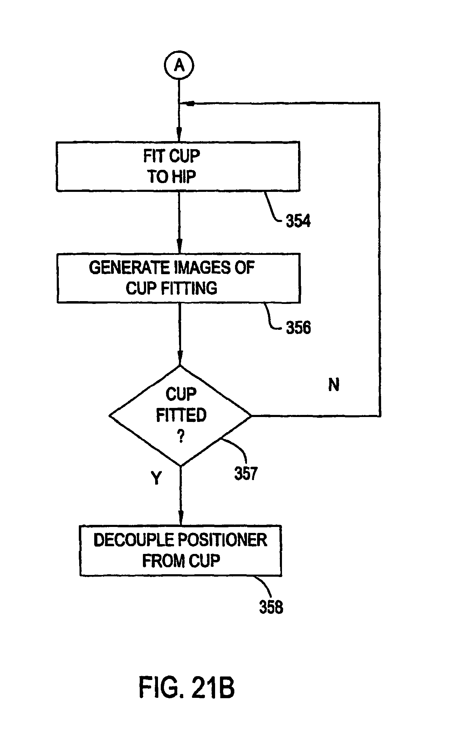

When, in the course of the surgical procedure it is time to mount the cup 290 to the hip, in a step 340 of Figures 21A and 21B, the cup is screw secured to positioner boss 316. In a step 342, the tracker 28 (Figure 1) is fitted to one of the prisms 332 or 334. Two prisms 332 and 334. are provided so the tracker can be placed in a location to best ensure that, during the procedure, the tracker is positioned so that the requisite line of sight path is established between the tracker 28 and localizer 30 (Figure 1). The providing of the two prisms 332 or 334 also facilitates optimal placing of the tracker for reasons of ergonomics.

In step 344, the tracker microcontroller 190 (Figure 15) reads the data from both the RFID of the prism to which the tracker 28 is attached and the cup RFID 302. As part of this process the data are forward through transceivers 198 (Figure 15) and 35 (Figure 1) to the system processor 32. The same general process described with respect to Figures 17A and 17B is used to ensure the data from each RFID are read and transmitted to the system processor 32 (Figure 1). System processor 32 then enters a reactive workflow protocol to further facilitate the performance of the surgical procedure. As part of this process the processor 32 reviews the data from the cup RFID 302 to determine the type of cup, step not illustrated. Then, based on the data previously acquired during the procedure, in a step 348, system processor 32 determines if the cup is appropriate for the procedure. For example, earlier in the procedure, a reamer 22 with a 48 mm diameter may have been used to form an opening in the bone for receiving the cup. In step 348, processor 32 determines if a complementary cup 290, a cup with the same diameter is now fitted to the positioner 292. If, in step 348 it is determined the cup is of a different size than the reamer, processor, in a step 350 causes an appropriate warning notice to be presented on display 36.

Thus, another feature is that the surgeon is provided with information if it appears a particular surgical instrument or implant appears inappropriate for use in a particular part of the procedure.

In step 348 it may be determined that the cup 290 is appropriate for fitting to the patient. If this determination is made, as part of the reactive workflow process, in step 352, processor 32 presents on the display 36 an image of the position of the cup 290 and positioner 292 relative to the surgical site. This image is based on: the data obtained from the localizer 40 regarding the position and orientation of the tracker 28; the data regarding the physical characteristics of the positioner from the RFID integral with one of the prisms 332 or 334; and the data from the cup RFID 302 describing the cup 290.

Also as part of step 352, additional information may be presented on display 36. these data include text messages that offer guidance for positioning the cup 290.

During the procedure, in step 354 of Figure 21B, positioner 292 is used to fit the cup 292. The real time monitoring of the changes in position and orientation of the tracker 28 is then used to determine by extension the exact position of the cup in the hip, step 356.

Once the cup 290 is properly positioned, step 357, the positioner 292 is decoupled from the cup, step 358. This action is accomplished by unscrewing the positioner boss 316 from the cup sleeve 298.

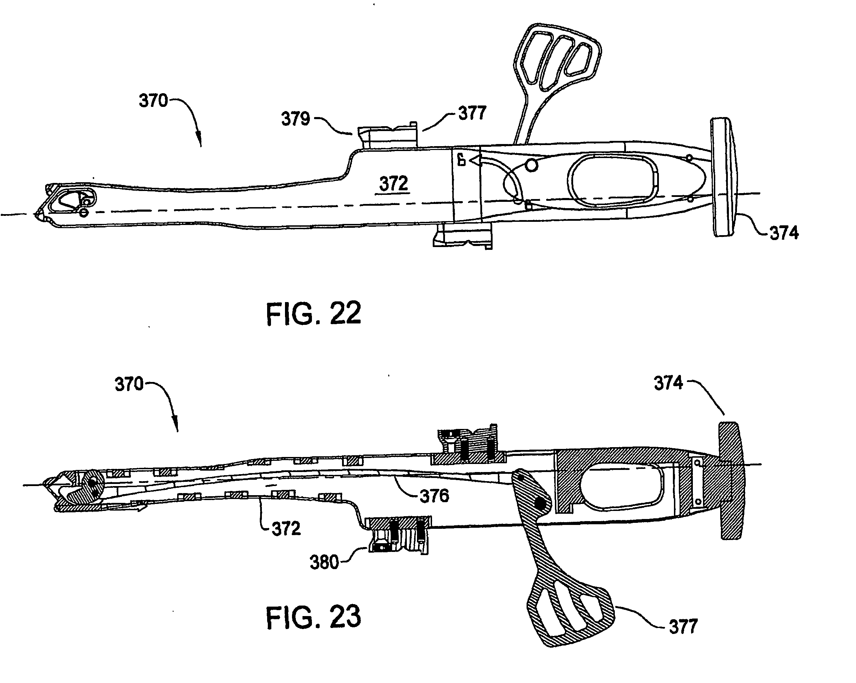

Figures 22 and 23 illustrate a broach handle 370. The broach handle 370 is used to hold a broach (not illustrated). The broach, sometimes called a rasp, is used to create a void space in the bone for insertion of a stem portion of an artificial joint. Broach handle 370 has an elongated flat body 372. A cap 374 is disposed over the proximal end of the body 372 to facilitate the holding of the handle 370 and the application of the force needed to press the broach against bone. A locking assembly 376, the exact structure and operation of which is not relevant to this invention, is fitted in the body 372. The locking assembly 376 contains the structural components of the handle 370 that releasably hold the broach to the handle. Locking assembly 376 includes a release lever 377 that is pivotally attached to the handle body 372. The release lever 377 is the manually actuated member of the locking assembly 376 that holds the broach in/releases the broach from the locked state.

Two prisms 379 and 380 are mounted to handle body 372. Each prism 379 and 380 includes its own RFID and coil as described above with respect to prisms 46, 332 and 334. Prisms 379 and 380, though, it will be observed are not symmetrically mounted to the handle body 372. Instead one prism, prism 379 is mounted to the body for receiving the tracker 28 when broach handle 370 is used in a procedure where the patient is placed in the lateral decubitus position. Examples of such procedures include, and are not limited to, posterior-lateral total hip arthroplasty (THA) and a mini-posterior-lateral THA. The second prism, prism 380, is mounted to the body 372 for receiving, a tracker when the broach handle 370 is used in a procedure. Examples of such procedures include, and are not limited to an anterior-lateral THA or a direct anterior THA. Thus, regardless of the type of procedure the broach handle is used to perform, the attached tracker is able to transmit data signals to the static transmitter.

While not illustrated, it should further be understood that the broaches used with handle 370 may be provided with their own RFIDs. These RFIDs are encapsulated in a manner similar to which reamer RFID 184 (Figure 15) or cup RFID 302 (Figure 20) is sealed. In these versions a sealed coil (not illustrated) is mounted to the distal end of the handle body 372. The coil serves as the antenna through which signals are exchanged with the RFID in the broach. Conductors that extend through handle body 372 connect the coil to the complementary coils disposed in the prisms 340 and 342.

In these versions, system processor 32 performs a reactive workflow process by first determining the geometry of the broach attached to the handle. Then in a step similar to step 348 (Figure 21A), the processor determines if the surgical site is set to accept the broach. Thus, if previous data indicates that a pilot bore of a specific size was formed in the bone, the system processor determines whether or not the broach attached to the handle is to large to be received in the pilot bore. Also as part of the evaluation step, system processor may evaluate if based on measurements of bone shape and dimensions, the expanded bore the broach is shaped to cut will too big for the surrounding bone.

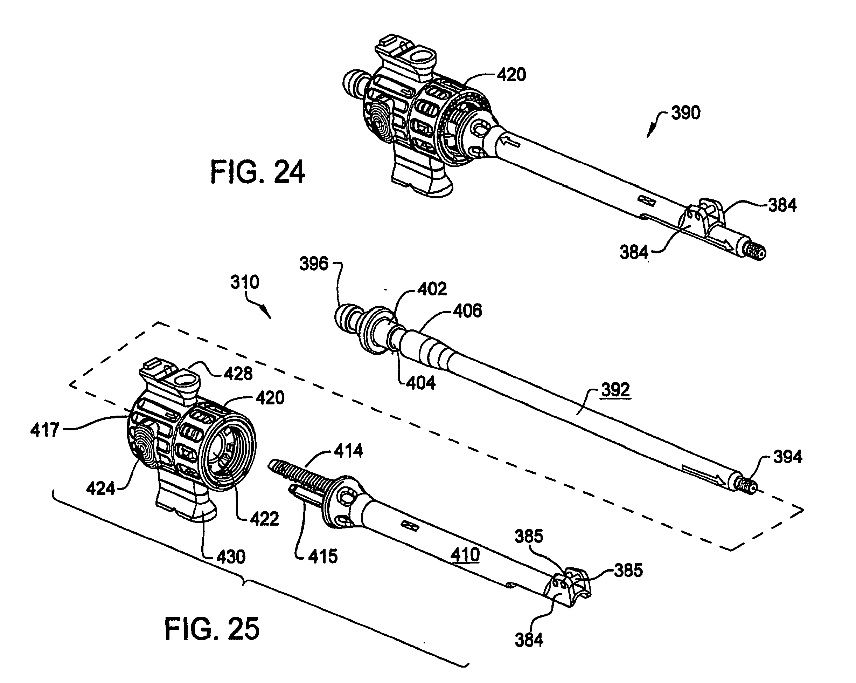

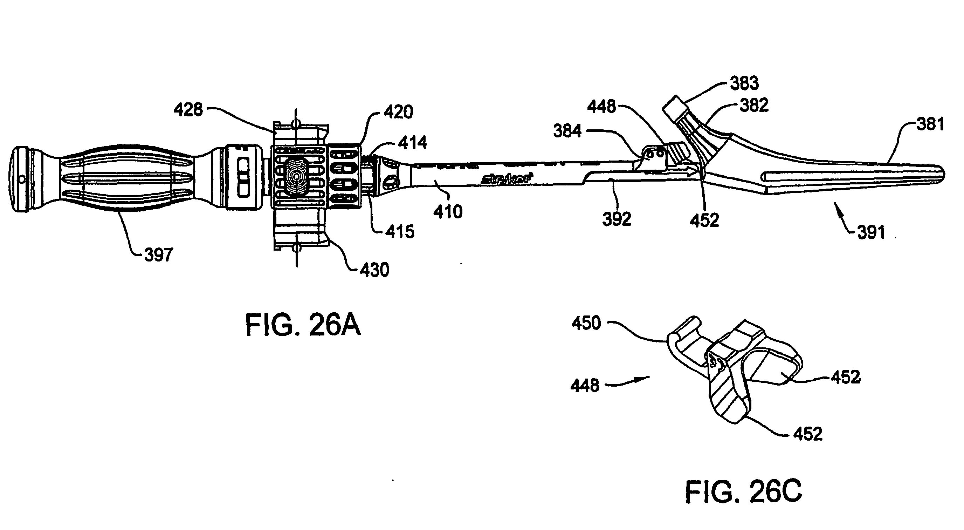

Figures 24-25 illustrate another surgical instrument, a stem inserter 390. Stem inserter 390 is used to insert a stem 391 (Figure 26A), another artificial joint component in position. Stem 391 includes a body 381. Stem body 381 is the portion of the stem inserted in the bone. A neck 382 extends upwardly and tapers inwardly from the body. A prism 383, which has circular cross sectional profile and an inward taper, extends upwardly from stem neck 382. Prism 383, which forms the head of the stem is the member to which a ball (not illustrated) complementary to the acetabular cup is fit. Stem 391, like numerous other implants, trial implants and surgical instruments, it will be observed, is asymmetrically shaped.

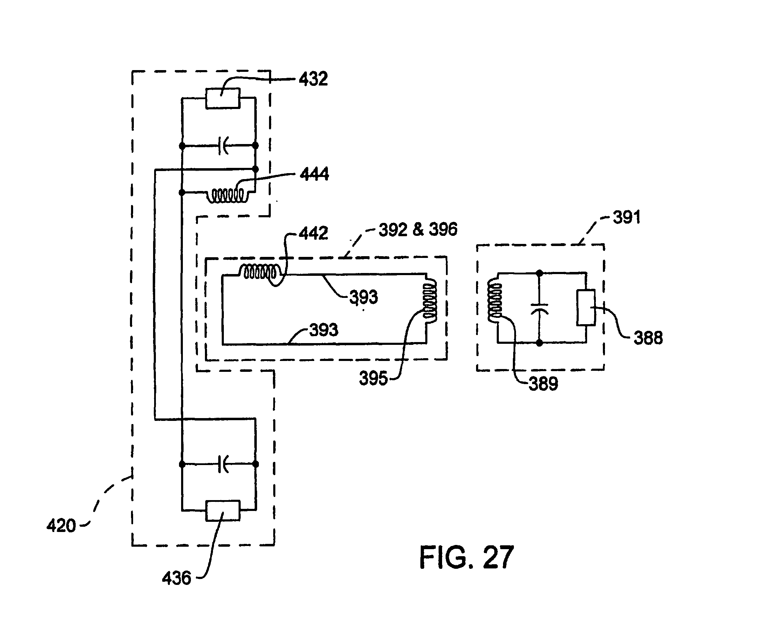

Returning to Figures 24 and 25 it can be seen that stem inserter 390 has a cylindrical shaft 392. A cylindrical boss 394 projects forward from the distal end of shaft 392. Boss 394, like positioner boss 316 (Figure 20), is provided with threading to facilitate the releasable coupling of a stem to the inserter 390. A coil 395 mounted in the distal of boss 396 provides for inductive signal exchange with a complementary coil 389 in the stem that is attached to the stem identifying RFID 388. Conductors 393 (Figure 27) extend through the shaft 392 and boss 394 to coil 395 or the conduits in which the conductors are seated. Not illustrated are the shaft and boss bores through which conductors 393 extend.

A knob 396 extends away from the proximal end of shaft 392. Knob 396, like positioner knob 312 (Figure 18), is designed to receive a grip or a handle 397. Distal to knob 396, shaft 392 is formed with a wide diameter circular flange 398. Distal to flange 398, shaft 392 has a head 402. Shaft head 402 has a diameter less than that of flange 398. Distal to head 402 the shaft has a neck 404 with a diameter less than that of the head. Neck 404 is located proximal to a shaft collar section 406. Collar section 406 has a diameter approximately equal to that of the head 402.

A generally sleeve shaped cannula 410 is rotating fitted over the shaft 392. Cannula 410 extends from a position proximal to the distal end of the shaft 392 to a position distal to shaft collar 406. While the inner and outer diameters of the cannula 410 are generally constant along the length, there is a proximal end base 412 that is outwardly flared. Cannula 410 is further formed so at the distal end there are two parallel, spaced apart, outwardly extending tabs 384. Two pins 385 extend between tabs 384.

Two prongs 414 and 415 extend proximally rearward from the proximal end of cannula base 412. Prongs 414 and 415 are symmetrically located around the center longitudinal axis of the cannula 410. Prong 414 is longer than prong 415. The outer surface of both prongs 414 and 415 is provided with threading (not identified).

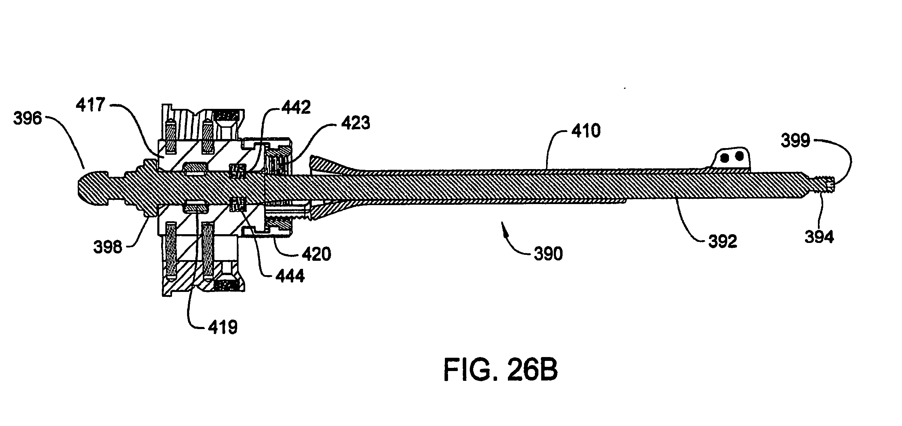

A barrel 417 is fitted to the cannula 410 and positioned over shaft head 402, neck 404 and collar 406. Barrel 417 is generally circular in shape, Barrel 417 is formed with a through bore 422 that allows the rotation movement of the barrel over the stem head 402, neck 404 and collar 406 sections. A lock assembly with a reverse biased release button 424 holds the barrel 417 in a fixed angular position relative to the longitudinal axis of the stem 392. More specifically, the lock assembly includes a plate 419 (partially seen in Figure 26B) disposed over the stem neck section 404. A spring, (not illustrated) holds plate 419 against the stem to, by extension, hold the barrel 417 in position. Depression of release button moves the lock plate 419 away from the stem so barrel 417 can be rotated.

While not illustrated, barrel 417 is also formed with two supplemental bores. The supplemental bores are parallel to bore 422 and symmetric to each other around the longitudinal axis of bore 422. Each of the supplemental bores is sized to slidably receive a separate one of the prongs 414 and 415. The supplemental bores are of unequal length such that only a single one of the bores can receive the longer of the two prongs, prong 415.

A ring 420 is rotatably mounted to the distal end of barrel 417. Ring 420 is formed with a threaded through bore 423. More particular the threading defining the ring bore 423 is positioned such that threading engages the outer surface threading of prongs 414 and 415.

Two prisms 428 and 430 are symmetrically disposed over barrel 417. Prisms 428 and 430 contain RFIDS 432 and 436, respectively and coils 434 and 438, respectively, seen in Figure 27. The capacitors connected across the coils are illustrated but not identified. The conductors that extend to stem coil 395 are electrically connected to the prisms integral with coils by two coils 442 and 444, best seen in Figures 26B and 27. Coil 442 is disposed in a recess in shaft collar 406. Coil 442 is the component to which the conductors 393 that extend to boss coil 395 are connected. Not shown is the protective ring disposed around coil 442 that protects the coil from the rigors of autoclave sterilization.

Coil 444 is disposed in barrel 417. More particularly, coil 444 is positioned to extend around the inner wall of the barrel 417 and positioned so as to subtend coil 442. Coil 444 is connected to coils 434 and 438 of, respectively, prisms 428 and 430. Again, a protective ring, not illustrated is disposed around the inner surface of coil 444 to protect the coil from the rigors of autoclave sterilization.

Stem inserter 390 also has an alignment yoke 448 best seen in Figures 26A and 26C. The alignment yoke is formed of plastic and has a J-shaped base 450. More particularly, the yoke base 450 is dimensioned to be releasably snap fit to pins 385 integral with cannula 410. The hook portion of the yoke base 385 extends over the proximal of the two pins. Two legs 452 extend outwardly from the yoke base 450. Legs 452 are symmetric with respect to longitudinal axis of the base 450. The legs 452 are spaced apart to define a space therebetween in which a portion of the complementary surgical implement can be received. In the depicted version, the legs are spaced apart a sufficient distance to receive a section of the stem neck 382.

Thus, inserter 390 is designed so that the barrel 417 can be rotated relative to the shaft 392 while still maintaining an electrical connection between prism coils 442 and 444 and shaft coil 395.

Also, inserter 390 is designed to be set in fixed spatial relationship, here an angular orientation, relative to the surgical implement, stem 391. More particularly, inserter 390 is configured for use by first fitting the barrel and ring sub assembly over the shaft 392 so that the proximal end of the barrel 417 seats against flange 398. Cannula 410 is fitted over the shaft 392 so that each prong 414 and 415 seats in the appropriate supplemental bore of the barrel. This step is accomplished by placing aligning prongs 414 and 415 with the supplemental bores and then rotating ring 420. The threading on the inside of ring bore 423 engages the prongs 414 and 415 to draw the cannula 410 proximally to the barrel 417.

Then, stem 391 is screw secured onto shaft boss 394. At this time the distal end of cannula 410 is spaced from the stem. Thus, the barrel and cannula sub assembly can freely rotate relative to the stem. This sub assembly is rotated to align yoke 448 with the stem neck 382. Ring 382 is then rotated. The rotation of ring 382 urges the cannula distally forward. Eventually this longitudinal displacement of the cannula results in the yoke legs 452 seating over the stem neck 382. As a consequence of the yoke seating around the stem neck 382, the cannula 410 and by extension barrel 417 and prisms 428 and 430 are in a specific known spatial (angular) relationship with the stem.

Thus it is provided a means to ensure that a tracked component, here the inserter 390 and a component attached to the tracked component are in a defined spatial relationship. This, in turn, ensures that the surgical navigation system is able to generate data that accurately indicates the position and orientation of the down lone attached component. Further, given the above example, this ensures such spatial position locking can be provided even if the attached component is, like stem 391, asymmetrically shaped.

It should be recognized that the implants are not just the actual implants permanently fitted to the patient.. They include trial implants.

An advantage of this feature is that when the surgeon switches trial implants, processor 32 is automatically provided with data indicating the trial implant with which the surgeon is presently working. Based on these data, the system automatically generates information indicating the location of the trial implant.

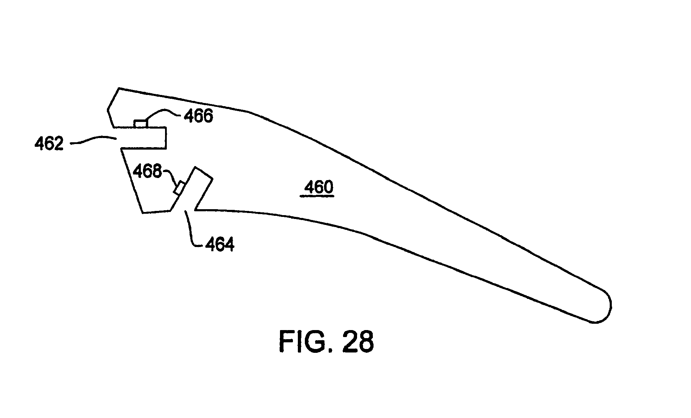

There may also be versions wherein it may be desirable to provide the cutting accessory or implant with plural RFIDs located at different locations. Such an assembly is represented diagrammatically in Figure 28. Here an implant 460 has two spaced apart bores 462 and 464. Each bore 462 and 464 serves as an opening wherein a handle can be positioned to manipulate the implant. Two bores 462 and 464 that are spaced apart from each other are provided to give the surgeon a choice of locations regarding where the handle is to be attached. This choice is desirable to accommodate individual surgeon preferences for manipulating the implant or viewing the implant 460 upon implementation. Coupling members, not illustrated, are disposed insider the bores 282 for holding the implant 460 to the handle.

In the above version, a separate RFID 466 and 468 is associated with each bore 462 and 464, respectively. The memory internal to each RFID 466 and 468 also contains an orientation field 470 (Figure 16). The data in the orientation field 470 indicates the orientation of the handle relative to the implant when the handle is fitted in the specific associated'bore 462 or 464.

These orientation data are part of the data that are read by the tracker 28 and forwarded to the processor 32. Based on the orientation data, the processor 32 generates a virtual image of the handle and implant assembly that accurately indicates the relative orientation of the handle and the implant. Cutting devices and other surgical components can similarly be provided with multiple coupling members and RFIDs that contain orientation data for specific to the complementary coupling members.

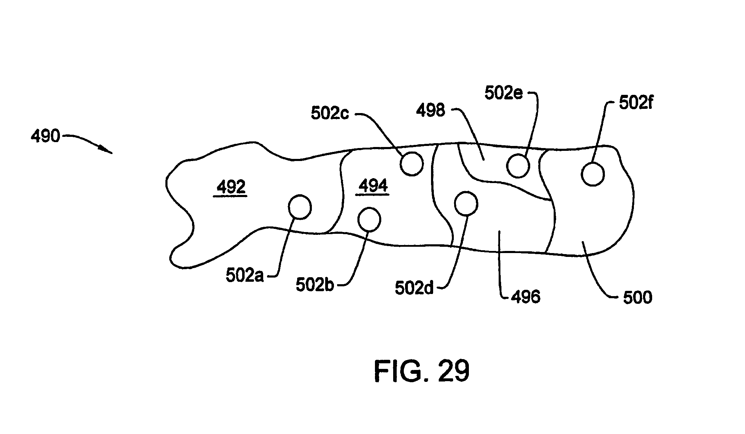

System 20 is also used to provide an inventory of bone and other tissue that may be temporarily removed and reinserted at the surgical site during the course of an operation. For example, as illustrated in Figure 29, as a result of a trauma, a bone 490 may fracture into different sections 492-500. In Figure 29, for reasons that will become apparent, sections 492 and 500 are sections that are not removed from the site of the trauma. Sections 494, 496 and 498 are bone sections, fragments, that, as part of the procedure to repair the bone 490, it is necessary to remove and then reattach. As long as the fragments 494, 496 and 498 are at least loosely together at the surgical site, the relative positions of the sections can be easily determined. However, once the bone fragments 494, 496 and 498 are removed from the surgical site, it may be difficult to determine the exact order in which they should be properly arranged.

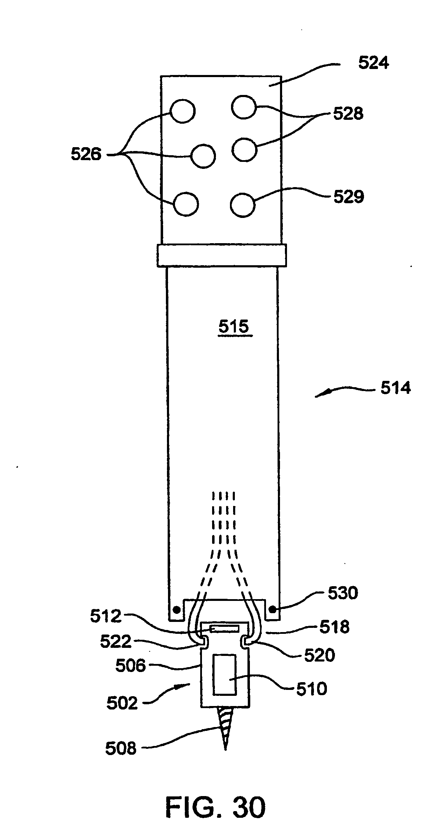

System 20 includes a set of pins 502a-f that mark the positions of the individual bone segments 492-500. For reasons discussed below, two pins, 502b and 502c, mark segment 294. As seen in Figure 30, each pin 502 consists of a generally cylindrical head 506. Extending downwardly from the head is a conically shaped shaft 508. The outer surface of shaft 508 is threaded, (threading not identified) to facilitate the screw securement of the pin 502 into a bone section. Alternatively, instead of being provided with a shaft, an exposed layer of adhesive material may be attached to the distal end of the pin head 506. The adhesive is of a type that adheres to bone and that upon application of a release force can be removed. Alternative methods of attaching the pin head 506 to a bone include simply providing shaft 508 with a pointed distal end that, upon the application of pressure can penetrate and lock into bone.

Internal to the pin head is an RFID 510 that is connected to a coil 512. While not illustrated, it should be understood that a tuning capacitor may be connected in parallel across the'RFID 510. The memory in RFID 510 contains a data field in which data are stored that identifies that associated pin 502 with specificity.

A handle 514 secures the pins 502 into the bone sections 492-500 before the removal of the bone fragments 494, 496 and 498 from the surgical site. Handle 514 includes an elongated shaft 515. A collet assembly extends out from the proximal end of the shaft 515. The collet assembly includes a plurality of legs 518 (two legs shown partially in solid and partially in phantom). Each leg 518 has a foot 320 that extends inwardly. Each collet foot 520 seats in a complementary indentation 522 formed in pin head 306. A lock and latch mechanism, also part of the collet assembly and not illustrated, urges the collet feet 520 towards the longitudinal center axis of the handle shaft 515. When the collet assembly is in this state, the collet feet 520 hold the pin 502 fast to the distal end of the handle 514.

A tracker 524 is integrally attached to the proximal end of the handle shaft 515. Illustrated are the location identifying LEDs 526, the data transmitting LEDs 328 and the data receiving photosensor 529. Tracker 524, while attached to shaft 515, is able to rotate relative to the shaft. A coil 530 is disposed in the distal end of the shaft 515. Coil 530 is positioned to inductively exchange signals with coil 512 integral with the pin 502.

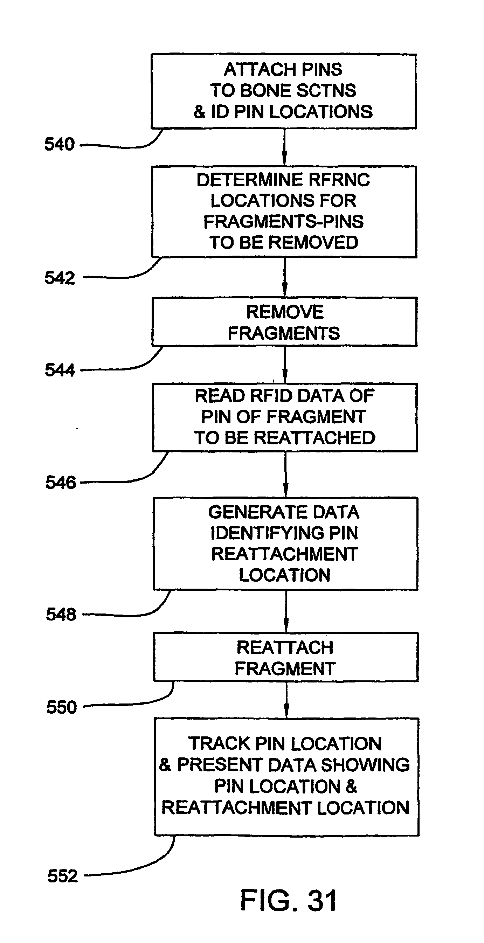

When system 20 is used to keep track of bone sections 492-500, handle 514 is used to attach the individual pins 502a-e, step 540 in Figure 31. Under the protocol, the handle 514 is used to drive the pin shaft secure the pin shaft into the bone section. The pins 502a-e are driven into the individual sections prior to the removal of the bone fragments 494, 496 and 498. Once each pin 502 is sufficient secured, as part of step 540, the tracker 524 is rotated relative to the handle shaft 515 so the LEDs 526 and 528 are directed to the localizer 30. The LEDs 526 provide the localizer data from which the position of the pin 30 is determined. As part of step 540, for each pin the data in the RFID 510 are transmitted through coils 312 and 330 to the tracker 524. The tracker 524 broadcasts these data through LEDs 328 to the localizer transceiver 35.

The localizer transceiver 35 forwards the data from LEDs 526 and 328 to the system processor 32. Based on these data, the system processor 32 assigns each pin 502 and the respective bone section 492-500 a specific location at the surgical site. Based on the data regarding the location of the pins 502a and 502f in the bone sections 492 and 500, respectively, that are not removed, the processor determines the locations where the pins 502b, c, d and e are to be returned, step 542. These are the pins attached to the bone fragments 494, 496 and 498 that, as part of the procedure, are removed from the surgical site. As part of step 542, this location identifying data are stored in a look-up table internal to the memory integral with the processor 32.

The bone sections to be removed, fragments 494, 496 and 498 are then removed, step 544. Handle 514 or other surgical instrument is employed to remove the fragments. The bone fragments 494, 496 and 498 are removed with the pins 502b, c, d and e still attached.

At the point in a procedure in which it is necessary to reattach a specific bone fragment 494, 496, or 498, the handle 514 is reattached to the pin 502b, 502d or 502e integral with the fragment. The data in the pin RFID 510 are read back into the system processor 32, step 546.

The processor 32 references the table established in memory of pin locations. Based on these data and the identity of the specific pin 502b, d, or e the processor provides information regarding the location at the surgical site at which the bone fragment attached to the surgical site should be reattached, step 548.

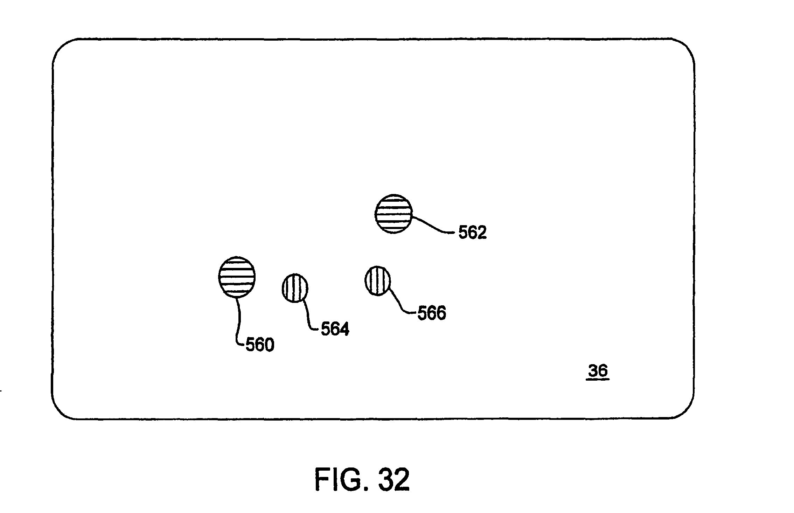

During the reattachment procedure, step 550, handle 514 is used to grasp the pin 502 so the pin-and-bone fragment is returned to the appropriate position. Based on the data indicating from where the pin 502 was when the bone fragment was removed, and the data regarding the location of the fixed points, the system processor 32 provides information regarding the exact location at the surgical site at which the bone fragment should be placed, step 352. As depicted in Figure 32, this information is often presented on the display 36 as in image indicating the target location for the pin, blue dot 560 (the location at which the pin should be located) and the actual location of the pin, red dot 564.

Thus, system 20, without wires, provides a means to both maintain a record of where, relative to each other removed body tissue is located and where, at the surgical site, the tissue is to be reattached.

In an alternative version of the above system, two or more pins 502 are attached to each bone fragment to be removed. This is depicted in Figure 29 wherein two pins 502b and 502c are attached to bone fragment 494. In step 542, the location of both pins 502b and 502c is determined and stored in the system processor. During the fragment reattachment process, steps 550 and 552, the location of both pins is tracked. Processor 32 causes display 36 to present images of both the target return and actual locations of each pin integral with fragment 494. Thus, in Figure 22, blue dots 560 and 562 may be the target return locations for pins, 502b and 30c, respectively. Red dots 564 and 566 are the actual locations of the pins.

This information allows the surgeon to determine both the correct location at which the fragment should be positioned and the proper orientation of the fragment.

In the above version, a handle with plural movable legs may be simultaneously locked to both pins 502b and 502c. Each leg contains a coil for reading the data associated with the complementary pin. Sensor provide data regarding the orientation of the legs relative to each other. Both the data read from the pin RFIDs and the data indicating the orientation of the legs is supplied through the associated tracker to the system processor 32. Based on the data describing the leg positions, using geometric formulas, the processor 32 is able to determine the relative positions of the pins to each other.

The foregoing description is directed to specific versions. It should be appreciated that other versions may have features different what has been described

For example, there is no requirement that in all versions, the transmitting/receiving heads used to exchange signals with the tracker be integral with the localizer. In some versions , these heads may be positioned at different locations.

In the described version, the tracker 28 emits energy and the source of the transmissions is monitored by the localizer 30. These functions may be reversed in some surgical navigation systems. For example, in a magnetic system, the tracker contains sensor that monitor the strength of magnetic fields that emanate from fixed sources. Based on the differences in the strengths of the measured fields, processors integral with the tracker or system processor determine tracker location and orientation. Thus, it should be understood that this invention is not limited to integration with one specific type of navigation system.

Similarly, there is no requirement that in all versions of the invention, RFIDs be employed as the devices with memories in which data describing the associated surgical component are stored. In some versions, the memory may be a bar code placed on the device. In these versions , readers integral with the tracker scan the bar code data. Other circuitry internal to the tracker converts the data into a form in which it can be broadcast to by the tracker to the associated static receiver. Alternatively, the data may be stored in other forms of memories that are read by optical scanning or other reading methods.

Electrical connectors in the tracker and complementary surgical components that require physical connection may be used as the signal transmitting/receiving heads. However, given the' presence of conductive fluids at surgical sites, this is not a preferred means of connection.

Also, while it is preferred that the tracker only have a single transmitting/receiving head for exchanging signals with the multiple downline components, coil 96 in the described embodiment, this is exemplary, not limiting. In some versions, the tracker may have multiple signal transmitting/receiving heads.

Also, in versions wherein the tracker is able to, through a single signal transmitting/receiving head obtain signals from multiple attached components, different protocols may be used to ensure that the data from the multiple memories are read. For example, in some versions of the invention, the RFID or other storage device associated with each class of component may be set to, in response to read request, be configured to write out the data after a specific delay period. Thus, in response to the tracker sending a basic identity interrogation signal, each component, at a different time, responds with a signal containing the identifying data. In this manner, the tracker microcontroller receives in a sequence, data that identifies each of the attached components.

Furthermore, it should be appreciated that the method set forth in the main description regarding how the decoder/encoder 202 responds to the receipt of simultaneous responses to the basic interrogation signal may change. The above method is employed only if the decoder/encoder, through other protocols, cannot respond to data collisions. In one alternative protocol, the decoder/encldoer, when collisions occur, may respond by repeatedly sending out basic interrogation signals. In response to the multiple sets of collided responses, the decoder/encoder may be able to determine the identity of the individual responding components.

In a variation of the primary described method for dealing with data collisions, resistors are series connected to the RFIDs associated with some surgical components. For example, it may be desirable to attach resistors with a high resistance to the RFIDs of the components that would be most distal to the decoder/encoder. Resistors with a lower resistance are attached to the RFIDs of the components intended for connection at an intermediate distance to the decoder/encoder. No resistors are attached to the RFIDs of the components normally located proximal to the decoder/encoder.

As a consequence of the above circuit arrangement, when the first basic interrogation signal is sent, the strengths of the signals associated with the RFIDs distal to the decoder/encoder are significantly attenuated. Consequently, there is essentially no likelihood these signals are read by the decoder/encoder during the time the reply signals to the first basic interrogation signal is received. Therefore, the decoder/encoder since it would initially only receive the basic identify reply from the RFID of the closest component, would immediately read the data from that RFID. Steps 238 and 239 could thus potentially be eliminated. Again, after the data from the closest RFID are read, the RFID is instructed to respond to the next basic interrogation. This would allow the data in the more distal RFIDs to be read.

Similar changes may be made in the mechanical structure of the features. For instance, in some versions of the invention, a secondary conduit may be formed in handle 44 to extend the length of the conduit. Conductors 106 may be disposed in this conduit. This conduit would thus eliminate the need to provide the outer sleeve. Also, a highly magnetically permeable sleeve may be fitted around a portion of bearing 58. Coil 104 may is then wound around this sleeve.

In the described version, the tracker is described as being attached to non-motorized components. This should not be considered limiting. For example, in some versions of the invention, the tracker may be removably attached to or integrally built into a power tool such as the power driver 26. In this version , a coil located in the power driver 26 is used to read the data in the surgical components attached to the power driver.

In these versions, sensors integral with the power tool provide data regarding the operating state of the tool. During the time periods the tool is actuated, the sensor data are transmitted through the tracker to the surgical navigation unit. The system processor 32 based on these data and the operational data read from the component RFID determines whether or not, for the specific attached component, the tool is being operated appropriately.

For example, the RFID integral with a specific reamer 22 in the operating parameter field 222 stores data indicating the maximum speed at which the reamer should be rotated. During the procedure, the power driver 26, through the attached tracker, provides system processor 32 with data indicating the speed at which the driver is being operated. If this speed is above the maximum reamer speed, processor 32 displays a warning message. Processor 32 may also, in a log, record that the reamer was driven at the excessive speed.

Also, there is no requirement that in all versions wherein RFID pins are used to track body fragments, the devices used to mount the pins also be used to provide the pin RFID data to the system processor 32. In some versions a first surgical tool may be used to mount the RFID pin. Then once a pin is mounted, a second pointer-with-tracker is locked to the pin. Once this component is so locked, the pointer-with-tracker is used to read and transmit the pin RFID data to the system processor 32 and generate data indicating the position of the pin.

It should be appreciated from the description of handle 514, that, in some versions, the tracker is integrally assembled into a first surgical component that both reads the data from the RFID integral with a second component and that manipulates the second component.

Alternative means to hold pins 502 to a handle may be employed. For example, the pin head 306 may be formed with geometric features such as threading. The distal end of the handle 514 may have a bore dimensioned to receive the pin head 306. The handle is formed with structural or geometric features in the bore that serve to securely and releasably hold the pin head in the bore.

Also, while the disclosed method of tracking body tissue describes how hard tissue, bone, can be tracked and then guided for proper return, this is only an example. This system and method may employ tags that are attached to other types of tissue, such as soft tissue to ensure that they are appropriately returned. In these versions, the tracking pins may be provided small spring feet. These feet are used to clamp around a section of tissue to be removed. Thus, if a tendon is removed and then reinstalled, by attaching these pins, the surgeon will have markers regarding where, upon reinstallation, certain sections of the tendon should be positioned.

Moreover, the type of data stored in the memory integral with the component is clearly a function of component type. For example, if the component is an RF ablation electrode, the RFID would store therein data describing the operating temperature of the electrode, the amount of current the electrode should draw and the type of cannula in which the electrode can be inserted.

It should likewise be appreciated that the means for encapsulating the data storage devices, the RFIDs, may be different from what has been described. In some versions a cap formed from a polymer may be fitted over the RFID in the reamer or the handle prism. In some versions, this cap may be formed from metal. In these versions , laser welding may be employed to seal the cap. This cap can also be formed from a ceramic. In still other versions, this cap, which may be three dimensional or in the form of a planar disk, is formed from combinations of two or more of the above materials.

Some processing functions performed by the system processor 32 may alternatively be performed by the tracker microcontroller 190. For example, based on the data received from the component RFIDs, the microcontroller may determine whether or not the associated surgical components can be used together and whether or not sterilization service or run time data indicates a particular component is ready for use. If the microprocessor 190 detects a fault state, it forwards an appropriate message to the system processor 32. Processor 32, in turn, causes an appropriate warning message to be presented on display 36.

Also, it should be appreciated that there is no limit to the types of surgical components, surgical implements, that can be provided with an RFID and employed in the reactive surgical and surgical navigation system. Other components that can be provided with RFIDs include: acetabular window trials; femoral reamers; femoral neck trials; femoral heads, including trial heads; and femoral implants. Surgical implements, including trial inserts and the actual implants themselves used for other joint replacement procedures may likewise be fitted with RFIDs so they can be used in the wireless reactive surgical and surgical navigation system.

Similarly, the information stored in the RFIDs integral with the components may be different from what has been described. Each RFID memory, in addition to storing data that identifies the component also has space in which a surgical log can be maintained. Each time the component is used in a surgery, the data in the log are updated to reflect the date of the surgery. A code identifying the patient on which the surgery was performed may also be written into the log. These data would make it possible to after the surgery, if necessary, locate the patient on whom the component was used.

Further, means other than inductive signal transfer may be used to transfer data through from the implement, the implant, the tissue forming instrument, through the surgical instrument to the tracker. In some versions, fiber optic cables are disposed in the instrument to facilitate the transmission of optical signals to the tracker. The tracker includes a complementary receiver able to receive these signals and forward them to the data transceiver 198.

Therefore, it is an object of the appended claims to cover all such variations and modifications that come within the scope of the invention as defined in claim 1.

## Claims

1. A tracker for a surgical navigation system, said tracker including:
a body (42);
a coupling unit (40) attached to the body for releasably securing the body to a surgical component,
at least one transducer (192) configured to transmit signals to or receive signals from the surgical navigation system so that the surgical navigation system is adapted to determine the position of the tracker and the surgical component,
a receiver (96) attached to the body or the coupling unit, said receiver adapted to receive signals from the surgical component to which the coupling unit is attached; and
a transmitter assembly (196, 198) attached to the body and is connected to the receiver, said transmitter assembly configured to receive the signals from said receiver and transmit the signals wirelessly to a remote receiver (35).

2. The tracker of Claim 1, wherein:
said receiver (96) is configured to be inductively coupled to a complementary transmitter integral with the surgical component.

3. The tracker of Claim 1, wherein:
said receiver is configured to receive light signals from the surgical component.

4. The tracker of Claims 1, 2 or 3, wherein:
said transmitter assembly is configured to transmit light signals to the remote receiver.

5. The tracker of Claims 1, 2 or 3, wherein:
said transmitter assembly is configured to transmit RF signals to the remote receiver.

6. The tracker of any one of Claims 1 to 5, wherein:
said receiver is disposed in the coupling unit so that said receiver can only exchange signals with the surgical component when the coupling unit engages the surgical component.

7. The tracker of any one of Claim 1 to 6, wherein:
the at least one transducer transmits or receives light-energy.

8. The tracker of any one of Claims 1 to 6, wherein:
the at least one transducer transmits or receives RF energy or EM energy or sonic energy.

9. The tracker of any one of Claims 1 to 8, wherein:
an inductor (210) and a capacitor (204) are connected to a decoder/encoder (202) for converting the signals received from said decoder/encoder (202) to sinusoidal signals and forwarding the sinusoidal signals to a coil (96) of the receiver, wherein the coil (96) is adapted to send signals.

10. The tracker according to any one of Claims 1 to 6, wherein:
the receiver (96) is formed by an electrical connector.

11. A surgical instrument for fitting a surgical implement, said instrument including:
the surgical navigation system tracker according to any one of Claims 1 to 10;
a body (118) having opposed proximal and distal ends;
a coupling assembly (63) attached to the distal end of said body configured to removably hold a surgical implement (22) to said body (118);
a first head (60) disposed in the distal end of said body (118) for contactlessly receiving signals from a memory (184) integral with the surgical implement (22);
a mounting block (46) attached to said body, said mounting block (46) configured to removably engage with the surgical navigation system tracker coupling assembly (40); and
a second head (82) disposed in said mounting block (46), said second head connected to said first head for receiving signals from the first head (60) and configured to contactlessly transmit the signals to the receiver in the tracker.

12. The surgical instrument of Claim 11, wherein:
said first head (60) is configured to inductively exchange signals with a head integral with the surgical implement memory (184);
and said second head (82) is configured to inductively exchange signals with the tracker receiver.

13. The surgical instrument of Claims 11 or 12 wherein:
a plurality of said mounting blocks are attached to said body at spaced apart locations along said body and each said mounting block has a separate said second head, each said second head being attached to said first head.

14. The surgical instrument of Claims 11, 12 or 13, wherein:
said mounting block is rotatingly attached to said body; and
an alignment unit extends distally from said mounting block to releasably abut the surgical implement to position the surgical implement and said mounting block in a spatial relationship.

15. A surgical instrument including:
the surgical navigation system tracker according to any one of Claims 1 to 10;
a body (392) for positioning a surgical implement (391), said body (392) having a distal end;
a coupling member (394) attached to the distal end of said body, said coupling member configured to releasably hold the surgical implement (391) to said body;
a mounting block (417) movably attached to said body (392), said mounting block shaped to removably engage with the surgical navigation system tracker coupling assembly;
an alignment unit (410) that extends from said mounting block to releasably abut the surgical implement (391) to position the surgical implement and said mounting block (417) in a select spatial relationship;
a memory (466, 468) attached to said body of said mounting block that stores data describing said surgical instrument; and
a transmitter in said mounting block (417) attached to said memory, said transmitter connected to said memory for contactlessly transmitting signals containing the data in said memory (466, 468) to the receiver in the tracker.

16. Surgical component, wherein:
the surgical component is coupled with the surgical navigation system tracker according to one of Claims 1 to 10.

17. Surgical component according to Claim 16, wherein:
the surgical component is an implant or surgical instrument.

## Patentansprüche

1. Nachverfolgungseinrichtung für ein chirurgisches Navigationssystem, wobei die Nachverfolgungseinrichtung umfasst:
einen Körper (42);
eine Kopplungseinheit (40), die am Körper zum lösbaren Sichern des Körpers an einem Chirurgie-Element angebracht ist,
zumindest einen Übertrager (192), der dazu ausgestaltet ist, Signale an das chirurgische Navigationssystem zu senden oder Signale von diesem zu empfangen, so dass das Navigationssystem dazu eingerichtet ist, die Position der Nachverfolgungseinrichtung und des Chirurgie-Elements zu bestimmen,
einen Empfänger (96), der am Körper oder an der Kopplungseinheit angebracht ist, wobei der Empfänger dazu eingerichtet ist, Signale von dem Chirurgie-Element zu empfangen, an dem die Kopplungseinheit angebracht ist; und
eine Sendeanordnung (196, 198), die am Körper angebracht ist und mit dem Empfänger verbunden ist, wobei die Sendeanordnung dazu ausgestaltet ist, Signale vom Empfänger zu empfangen und die Signale drahtlos zu einem entfernten Empfänger (35) zu senden.

2. Nachverfolgungseinrichtung nach Anspruch 1, wobei:
der Empfänger (96) dazu ausgestaltet ist, mit einem komplementären Sender induktiv gekoppelt zu werden, der mit dem Chirurgie-Element integral ausgebildet ist.

3. Nachverfolgungseinrichtung nach Anspruch 1, wobei:
der Empfänger dazu ausgestaltet ist, Lichtsignale von dem Chirurgie-Element zu empfangen.

4. Nachverfolgungseinrichtung nach den Ansprüchen 1, 2 oder 3, wobei:
die Sendeanordnung dazu ausgestaltet ist, Lichtsignale an den entfernten Empfänger zu senden.

5. Nachverfolgungseinrichtung nach den Ansprüchen 1, 2 oder 3, wobei:
die Sendeanordnung dazu ausgestaltet ist, HF-Signale an den entfernten Empfänger zu senden.

6. Nachverfolgungseinrichtung nach einem der Ansprüche 1 bis 5, wobei:
der Empfänger in der Kopplungseinheit angeordnet ist, so dass der Empfänger lediglich dann Signale mit dem Chirurgie-Element austauschen kann, wenn die Kopplungseinheit mit dem Chirurgie-Element zusammen wirkt.

7. Nachverfolgungseinrichtung nach einem der Ansprüche 1 bis 6, wobei:
der zumindest eine Übertrager Lichtenergie sendet oder empfängt.

8. Nachverfolgungseinrichtung nach einem der Ansprüche 1 bis 6, wobei:
der zumindest eine Übertrager HF-Energie oder EM-Energie oder Schallenergie sendet oder empfängt.

9. Nachverfolgungseinrichtung nach einem der Ansprüche 1 bis 8, wobei:
eine Induktivität (210) und ein Kondensator (204) an einer Decodiereinrichtung/Codiereinrichtung (202) angeschlossen sind, um die von der Decodiereinrichtung/Codiereinrichtung (202) empfangenen Signale in sinusförmige Signale umzuwandeln und um die sinusförmigen Signale an eine Spule (96) des Empfängers weiterzuleiten, wobei die Spule (96) dazu ausgebildet ist, die Signale zu senden.

10. Nachverfolgungseinrichtung nach einem der Ansprüche 1 bis 6, wobei der
Empfänger (96) durch einen elektrischen Verbinder gebildet ist.

11. Chirurgisches Instrument zum Aufnehmen eines chirurgischen Utensils, wobei das Instrument umfasst:
die Nachverfolgungseinrichtung für ein chirurgisches Navigationssystem nach einem der Ansprüche 1 bis 10;
einen Körper (118) mit einem proximalen und einem entgegengesetzten distalen Ende;
eine Kopplungsanordnung (63), die am distalen Ende des Körpers angebracht ist und dazu ausgestaltet ist, ein chirurgisches Utensil (22) am Körper (118) lösbar zu halten;
einen ersten Kopf (60), der im distalen Ende des Körpers (118) zum berührungslosen Empfangen von Signalen von einem Speicher (184) angeordnet ist, der mit dem chirurgischen Utensil (22) integral ausgebildet ist;
einen Anbringungsblock (46), der am Körper angeordnet ist, wobei der Anbringungsblock (46) dazu ausgestaltet ist, mit der Kopplungsanordnung (40) der Nachverfolgungseinrichtung für das chirurgische Navigationssystem lösbar zusammenzuwirken; und
einen zweiten Kopf (82), der im Anbringungsblock (46) angeordnet ist, wobei der zweite Kopf mit dem ersten Kopf zum Empfangen von Signalen vom ersten Kopf (60) verbunden ist und dazu ausgestaltet ist, die Signale zum Empfänger in der Nachverfolgungseinrichtung berührungslos zu senden.

12. Chirurgisches Instrument nach Anspruch 11, wobei:
der erste Kopf (60) dazu ausgestaltet ist, Signale mit einem Kopf induktiv auszutauschen, der integral mit dem Speicher (184) des chirurgischen Utensils ausgebildet ist;
und der zweite Kopf (82) dazu ausgestaltet ist, Signale mit dem Empfänger der Nachverfolgungseinrichtung induktiv auszutauschen.

13. Chirurgisches Instrument nach den Ansprüchen 11 oder 12, wobei:
eine Mehrzahl der Anbringungsblöcke am Körper an voneinander beabstandeten Orten entlang des Körpers angeordnet ist und jeder Anbringungsblock einen separaten zweiten Kopf aufweist, wobei jeder zweite Kopf am ersten Kopf angebracht ist.

14. Chirurgisches Instrument nach den Ansprüchen 11, 12 oder 13, wobei:
der Anbringungsblock drehbar am Körper angeordnet ist; und
sich eine Ausrichtungseinheit distal vom Anbringungsblock erstreckt, um das chirurgische Utensil lösbar anzulegen, um das chirurgische Utensil und den Anbringungsblock in einer räumlichen Beziehung zu positionieren.

15. Chirurgisches Instrument, mit:
der Nachverfolgungseinrichtung für ein chirurgisches Navigationssystem nach einem der Ansprüche 1 bis 10;
einem Körper (392) zum Positionieren eines chirurgischen Utensils (391), wobei der Körper (392) ein distales Ende aufweist;
einem Kopplungselement (394), das am distalen Ende des Körpers befestigt ist, wobei das Kopplungselement dazu ausgestaltet ist, das chirurgische Utensil (391) am Körper lösbar zu halten;
einem Anbringungsblock (417), der beweglich am Körper (392) angeordnet ist, wobei der Anbringungsblock so geformt ist, dass er mit der Kopplungsanordnung der Nachverfolgungseinrichtung für das chirurgische Navigationssystem lösbar zusammenwirkt;
einer Ausrichtungseinheit (410), die sich von dem Anbringungsblock erstreckt, um das chirurgische Utensil (391) lösbar anzulegen, um das chirurgische Utensil und den Anbringungsblock (417) in einer ausgewählten räumlichen Beziehung zu positionieren;
einem Speicher (466, 468), der an dem Körper des Anbringungsblocks befestigt ist und der Daten speichert, die das chirurgische Instrument beschreiben; und
einem Sender im Anbringungsblock (417), der am Speicher angeordnet ist, wobei der Sender mit dem Speicher verbunden ist, um berührungslos Signale, die die Daten im Speicher (466, 468) enthalten, an den Empfänger in der Nachverfolgungseinrichtung zu senden.

16. Chirurgie-Element, wobei
das Chirurgie-Element mit der Nachverfolgungseinrichtung nach einem der Ansprüche 1 bis 10 des chirurgischen Navigationssystems verbunden ist.

17. Chirurgie-Element nach Anspruch 16, wobei
das Chirurgie-Element ein Implantat oder ein chirurgisches Instrument ist.

## Revendications

1. Dispositif de poursuite pour un système de navigation chirurgicale, ledit dispositif de poursuite comprenant :
un corps (42) ;
une unité de raccordement (40) reliée au corps pour fixer de manière détachable le corps à un composant chirurgical,
au moins un transducteur (192) configuré pour émettre des signaux vers le système de navigation chirurgicale ou en recevoir en provenance de celui-ci, de sorte que le système de navigation chirurgicale est conçu pour déterminer la position du dispositif de poursuite et du composant chirurgical,
un récepteur (96) relié au corps ou à l'unité de raccordement, ledit récepteur conçu pour recevoir des signaux en provenance du composant chirurgical auquel l'unité de raccordement est reliée ; et
un assemblage formant émetteur (196, 198) relié au corps et est raccordé au récepteur, ledit assemblage formant émetteur configuré pour recevoir les signaux provenant dudit récepteur et émettre sans fil les signaux vers un récepteur à distance (35).

2. Dispositif de poursuite selon la revendication 1, dans lequel :
ledit récepteur (96) est configuré pour être couplé par induction à un émetteur complémentaire solidaire du composant chirurgical.

3. Dispositif de poursuite selon la revendication 1, dans lequel :
ledit récepteur est configuré pour recevoir des signaux lumineux en provenance du composant chirurgical.

4. Dispositif de poursuite selon les revendications 1, 2 ou 3, dans lequel :
ledit assemblage formant émetteur est configuré pour émettre des signaux lumineux vers le récepteur à distance.

5. Dispositif de poursuite selon les revendications 1, 2 ou 3, dans lequel :
ledit assemblage formant émetteur est configuré pour émettre des signaux RF vers le récepteur à distance.

6. Dispositif de poursuite selon l'une quelconque des revendications 1 à 5, dans lequel :
ledit récepteur est disposé dans l'unité de raccordement, de sorte que ledit récepteur peut seulement échanger des signaux avec le composant chirurgical lorsque l'unité de raccordement coopère avec le composant chirurgical.

7. Dispositif de poursuite selon l'une quelconque des revendications 1 à 6, dans lequel :
ledit au moins un transducteur émet ou reçoit de l'énergie lumineuse.

8. Dispositif de poursuite selon l'une quelconque des revendications 1 à 6, dans lequel :
ledit au moins un transducteur émet ou reçoit de l'énergie radioélectrique ou de l'énergie électromagnétique ou de l'énergie sonore.

9. Dispositif de poursuite selon l'une quelconque des revendications 1 à 8, dans lequel :
une bobine d'inductance (210) et un condensateur (204) sont connectés à un décodeur-codeur (202) pour convertir les signaux reçus en provenance dudit décodeur-codeur (202) en signaux sinusoïdaux et réacheminer les signaux sinusoïdaux vers une bobine (96) du récepteur, dans lequel la bobine (96) est conçue pour envoyer des signaux.

10. Dispositif de poursuite selon l'une quelconque des revendications 1 à 6, dans lequel :
le récepteur (96) est constitué d'un connecteur électrique.

11. Instrument chirurgical pour adapter un outil chirurgical, ledit instrument comprenant :
le dispositif de poursuite pour système de navigation chirurgicale selon l'une quelconque des revendications 1 à 10 ;
un corps (118) présentant des extrémités proximale et distale opposées ;
un assemblage de raccordement (63) relié à l'extrémité distale dudit corps, configuré pour maintenir de manière amovible un outil chirurgical (22) sur ledit corps (118) ;
une première tête (60) disposée dans l'extrémité distale dudit corps (118) pour recevoir sans contact des signaux en provenance d'une mémoire (184) solidaire de l'outil chirurgical (22) ;
un bloc de montage (46) relié audit corps, ledit bloc de montage (46) étant configuré pour coopérer de manière amovible avec l'assemblage de raccordement du dispositif de poursuite pour système de navigation chirurgicale (40) ; et
une seconde tête (82) disposée dans ledit bloc de montage (46), ladite seconde tête étant raccordée à ladite première tête pour recevoir des signaux en provenance de la première tête (60) et étant configurée pour émettre sans contact les signaux vers le récepteur dans le dispositif de poursuite.

12. Instrument chirurgical selon la revendication 11, dans lequel :
ladite première tête (60) est configurée pour échanger par induction des signaux avec une tête solidaire de la mémoire (184) de l'outil chirurgical ;
et ladite seconde tête (82) est configurée pour échanger par induction des signaux avec le récepteur du dispositif de poursuite.

13. Instrument chirurgical selon la revendication 11 ou 12, dans lequel :
une pluralité desdits blocs de montage est reliée audit corps à des positions espacées les unes des autres le long dudit corps et chacun desdits blocs de montage comporte l'une séparée desdites secondes têtes, chacune desdites secondes têtes étant reliée à ladite première tête.

14. Instrument chirurgical selon les revendications 11, 12 ou 13, dans lequel :
ledit bloc de montage est relié en rotation audit corps ; et
une unité d'alignement s'étend distalement à partir dudit bloc de montage pour venir en butée de manière détachable sur ledit outil chirurgical afin de positionner l'outil chirurgical et ledit bloc de montage dans une relation spatiale.

15. Instrument chirurgical comprenant :
le dispositif de poursuite pour système de navigation chirurgicale selon l'une quelconque des revendications 1 à 10 ;
un corps (392) pour positionner un outil chirurgical (391), ledit corps (392) présentant une extrémité distale ;
un élément de raccordement (394) relié à l'extrémité distale dudit corps, ledit élément de raccordement étant configuré pour maintenir de manière détachable l'outil chirurgical (391) sur ledit corps ;
un bloc de montage (417) relié de manière mobile audit corps (392), ledit bloc de montage étant formé pour coopérer de manière amovible avec l'assemblage de raccordement du dispositif de poursuite pour système de navigation chirurgicale ;
une unité d'alignement (410) qui s'étend à partir dudit bloc de montage pour venir en butée de manière détachable sur l'outil chirurgical (391) afin de positionner l'outil chirurgical et ledit bloc de montage (417) dans une relation spatiale choisie ;
une mémoire (466, 468) reliée audit corps dudit bloc de montage, qui stocke des données décrivant ledit instrument chirurgical ; et
un émetteur dans ledit bloc de montage (417) relié à ladite mémoire, ledit émetteur étant raccordé à ladite mémoire pour émettre sans contact des signaux contenant les données de ladite mémoire (466, 468) vers le récepteur dans le dispositif de poursuite.

16. Composant chirurgical, dans lequel :
le composant chirurgical est raccordé au dispositif de poursuite pour système de navigation chirurgicale selon l'une des revendications 1 à 10.

17. Composant chirurgical selon la revendication 16, dans lequel :
le composant chirurgical est un implant ou un instrument chirurgical.
